(19) Europäisches Patentamt
European Patent Office
Office européen des brevets

(11) EP 1 244 660 B1

# (12) EUROPEAN PATENT SPECIFICATION

(45) Date of publication and mention of the grant of the patent:
**22.03.2006 Bulletin 2006/12**

(21) Application number: **00987932.1**

(22) Date of filing: **19.12.2000**

(51) Int Cl.:
***C07D 471/04*** (2006.01) ***A61K 31/5025*** (2006.01)

(86) International application number:
**PCT/SE2000/002607**

(87) International publication number:
**WO 2001/047924 (05.07.2001 Gazette 2001/27)**

## (54) METHODS AND COMPOSITIONS FOR THE TREATMENT OF PAIN

METHODEN UND ZUSAMMENSETZUNGEN ZUR BEHANDLUNG VON SCHMERZEN

PROCEDES ET COMPOSITIONS POUR LE TRAITEMENT DE LA DOULEUR

(84) Designated Contracting States:
**AT BE CH CY DE DK ES FI FR GB GR IE IT LI LU MC NL PT SE TR**

(30) Priority: **23.12.1999 US 171906 P**
**29.09.2000 US 236881 P**

(43) Date of publication of application:
**02.10.2002 Bulletin 2002/40**

(73) Proprietor: **AstraZeneca AB**
**151 85 Södertälje (SE)**

(72) Inventors:
- **URBANEK, Rebecca Ann**
**AstraZeneca**
**Wilmington, DE 19850-5437 (US)**
- **BARE, Thomas, Michael**
**West Chester, PA 19832 (US)**
- **BROWN, Dean Gordon**
**AstraZeneca**
**Wilmington, DE 19850-5437 (US)**
- **XIAO, Wenhua**
**AstraZeneca**
**Wilmington, DE 19850-5437 (US)**
- **STEELMAN, Gary Banks**
**AstraZeneca**
**Wilmington, DE 19850-5437 (US)**
- **MURPHY, Megan**
**AstraZeneca**
**Wilmington, DE 19850-5437 (US)**
- **HORCHLER, Carey Lynn**
**AstraZeneca**
**Wilmington, DE 19850-5437 (US)**

(56) References cited:
**WO-A1-95/11244** **WO-A1-96/15127**

EP 1 244 660 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

### Field of the Invention

This invention relates to the treatment or prevention of pain or nociception.

### Related Art

Pain is a sensory experience distinct from sensations of touch, pressure, heat and cold. It is often described by sufferers by such terms as bright, dull, aching, pricking, cutting or burning and is generally considered to include both the original sensation and the reaction to that sensation. This range of sensations, as well as the variation in perception of pain by different individuals, renders a precise definition of pain difficult, however, many individuals suffer with severe and continuous pain.

Pain that is caused by damage to neural structures is often manifest as a neural supersensitivity or hyperalgesia and is termed "neuropathic" pain. Pain can also be "caused" by the stimulation of nociceptive receptors and transmitted over intact neural pathways, such pain is termed "nociceptive" pain.

The level of stimulation at which pain becomes noted is referred to as the "pain threshold." Analgesics are pharmaceutical agents which relieve pain by raising the pain threshold without a loss of consciousness. After administration of an analgesic drug a stimulus of greater intensity or longer duration is required before pain is experienced. In an individual suffering from hyperalgesia an analgesic drug may have an anti-hyperalgesic effect. In contrast to analgesics, agents such as local anaesthetics block transmission in peripheral nerve fibers thereby blocking awareness of pain. General anaesthetics, on the other hand, reduce the awareness of pain by producing a loss of consciousness.

Tachykinin antagonists have been reported to induce antinociception in animals, which is believed to be analogous to analgesia in man (Maggi et al, J. Auton. Pharmacol. (1993) 13, 23-93). In particular, non-peptide NK-1 receptor antagonists have been shown to produce such analgesia. For example, the NK-1 receptor antagonist RP 67,580 produced analgesia with potency comparable to that of morphine (Garret et al, Proc. Natl. Acad. Sci. USA (1993) 88, 10208-10212).

The opioid analgesics are a well-established class of analgesic agents with morphine-like actions. Synthetic and semi-synthetic opioid analgesics are derivatives of five chemical classes of compound: phenanthrenes; phenylheptylamines; phenylpiperidines; morphinans; and benzomorphans. Pharmacologically these compounds have diverse activities, thus some are strong agonists at the opioid receptors (e.g. morphine); others are moderate to mild agonists (e.g. codeine); still others exhibit mixed agonist-antagonist activity (e.g. nalbuphine); and yet others are partial agonists (e.g. nalorphine). Whilst an opioid partial agonist such as nalorphine, (the N-alkyl analogue of morphine) will antagonize the analgesic effects of morphine, when given alone it can be a potent analgesic in its own right.

Of all of the opioid analgesics, morphine remains the most widely used, but, in addition to its therapeutic properties, it has a number of drawbacks including respiratory depression, decreased gastrointestinal motility (resulting in constipation), nausea and vomiting. Tolerance and physical dependence also limit the clinical uses of opioid compounds.

Aspirin and other salicylate compounds are frequently used in treatment to interrupt amplification of the inflammatory process in rheumatoid diseases and arthritis and temporarily relieve the pain. Other drug compounds used for these purposes include phenylpropionic acid derivatives such as Ibuprofen and Naproxen, Sulindac, phenyl butazone, corticosteroids, antimalarials such as chloroquine and hydroxychloroquine sulfate, and fenemates (J. Hosp. Pharm., 36: 622 (May 1979)). These compounds, however, are ineffective for neuropathic pain.

Available therapies for pain also have drawbacks. Some therapeutic agents require prolonged use before an effect is experienced by the patient. Other existing drugs have serious side effects in certain patients, and subjects must be carefully monitored to ensure that any side effects are not unduly threatening. Most existing drugs provide only temporary relief from pain and must be taken consistently on a daily or weekly basis. With disease progression the amount of medication needed to alleviate the pain often increases, thus increasing the potential for adverse side effects.

NMDA receptors are defined by the binding of N-methyl-D-aspartate (NMDA) comprise a receptor/ion channel complex with several different identified binding domains. NMDA itself is a molecule structurally similar to glutamate (Glu) which binds at the glutamate binding suite and is highly selective and potent in activating the NMDA receptor (Watkins (1987); Olney (1989)).

Many compounds are known that bind at the NMDA/Glu binding site (for example CPP, DCPP-ene, CGP 40116, CGP 37849, CGS 19755, NPC 12626, NPC 17742, D-AP5, D-AP7, CGP 39551, CGP-43487, MDL-100,452, LY-274614, LY-233536, and LY233053). Other compounds, referred to as non-competitive NMDA antagonists, bind at other sites in the NMDA receptor complex (examples are phencyclidine, dizocilpine, ketamine, tiletamine, CNS 1102, dextromethorphan, memantine, kynurenic acid, CNQX, DNQX, 6,7-DCQX, 6,7-DCHQC, R(+)-HA-966, 7-chloro-kynurenic acid, 5,7-DCKA, 5-iodo-7-chloro-kynurenic acid, MDL-28,469, MDL-100,748, MDL-29,951, L-689,560, L-687,414, ACPC, ACPCM, ACPCE, arcaine, diethylenetriamine, 1,10-diaminodecane, 1,12-diaminododecane, ifenprodil, and SL-82.0715). These compounds have been extensively reviewed by Rogawski (1992) and Massieu et. al., (1993), and articles cited therein.

In addition to its physiological function, glutamate (Glu) can be neurotoxic. Glu neurotoxicity is referred to as "excitotoxicity" because the neurotoxic action of Glu, like its beneficial actions, is mediated by an excitatory process (Olney (1990); Choi (1992)). Normally, when Glu is released at a synaptic receptor, it binds only transiently and is then rapidly removed from the receptor by a process that transports it back into the cell. Under certain abnormal conditions, including stroke, epilepsy and CNS trauma, Glu uptake fails and Glu accumulates at the receptor resulting in a persistent excitation of electrochemical activity that leads to the death of neurons that have Glu receptors. Many neurons in the CNS have Glu receptors, so excitotoxicity can cause an enormous amount of CNS damage.

Acute excitotoxicity injury can occur as a result of ischemic events, hypoxic events, trauma to the brain or spinal cord, certain types of food poisoning which involve an excitotoxic poison such as domoic acid, and seizure-mediated neuronal degeneration, which can result from persistent epileptic seizure activity (status epilepticus). A large body of evidence has implicated the NMDA receptor as one receptor subtype through which Glu mediates a substantial amount of CNS injury, and it is well established that NMDA antagonists are effective in protecting CNS neurons against excitotoxic degeneration in these acute CNS injury syndromes (Choi (1988); Olney (1990)).

In addition to neuronal damage caused by acute insults, excessive activation of Glu receptors may also contribute to more gradual neurodegenerative processes leading to cell death in various chronic neurodegenerative diseases, including Alzheimer's disease, amyotrophic lateral sclerosis, AIDS dementia, Parkinson's disease and Huntington's disease (Olney (1990)). It is generally considered that NMDA antagonists may prove useful in the therapeutic management of such chronic diseases.

In the 1980's it was discovered that PCP (also known as "angel dust") acts at a "PCP recognition site" within the ion channel of the NMDA Glu receptor. PCP acts as a non-competitive antagonist that blocks the flow of ions through the NMDA ion channel. More recently it has become evident that drugs which act at the PCP site as non-competitive NMDA antagonists are likely to have psychotomimetic side effects. Further, it is now recognized that certain competitive and non-competitive NMDA antagonists can cause similar pathomorphological effects in rat brain (Olney et al., (1991); Hargreaves et. al., (1993)). Such compounds also have psychotomimetic effects in humans (Kristensen et. al., (1992); Herrling (1994); Grotta (1994)).

The glycine binding site of the NMDA receptor complex is distinguishable from the Glu and PCP binding sites. Also, it has recently been discovered that NMDA receptors occur as several subtypes which are characterized by differential properties of the glycine binding site of the receptor. Many compounds that bind at the NMDA receptor glycine site, useful for the treatment of stroke and neurodegenerative conditions, have been described in U.S. Patents 5,604,227; 5,733,910; 5,599,814; 5,593,133; 5,744,471; 5,837,705 and 6,103,721.

## Summary of the Invention

1. A compound selected from:

7-Chloro-4-hydroxy-2-(2,4,6-trimethylphenyl)-1,2,5,10-tetrahydropyridazino[4,5-*b*]quinoline-1,10-dione;
7,9-Dichloro-4-hydroxy-2-(4-methoxy-2-methylphenyl)-1,2,5,10-tetrahydropyridazino[4,5-*b*]quinoline-1,10-dione;
Methyl 4-[(7-chloro-4-hydroxy-1,10-dioxo-2,5-dihydropyridazino[4,5-b]quinolin-2-yl)methyl]benzoate;
7-Chloro-4-hydroxy-2-(4-*N*-furan-2-ylmethyl)benzamidylmethyl-1,2,5,10-tetrahydropyridazino[4,5-*b*]quinoline-1,10-dione;
7-Chloro-4-hydroxy-2-(4-dimethylaminobenzyl)-1,2,5,10-tetrahydropyridazino[4,5-*b*]quinoline-1,10-dione;
7-Chloro-4-hydroxy-2-(benzimidazol-5-ylmethyl)-1,2,5,10-tetrahydropyridazino[4,5-*b*]quinoline-1,10-dione;
7-Chloro-4-hydroxy-2-(4-hydroxy-3-methyl-benzyl)-1,2,5,10-tetrahydropyridazino[4,5-*b*]quinoline-1,10-dione;
7-Chloro-4,10-dihydroxy-2-[(2-methylthiophenyl)methyl]-2-hydropyridazino[4,5-*b*]quinoline-1-one;
7-Chloro-2-(4-(2,6-dimethoxy)methylbenzoate)-4-hydroxy-1,2,5,10-tetrahydropyridazino[4,5-*b*]quinoline-1,10-dione;
Methyl 3-[(7-chloro-4-hydroxy-1,10-dioxo-2,5-dihydropyridazino[4,5-*b*]quinolin-2-yl)methyl]benzoate;
7-Chloro-4-hydroxy-2-(4-dimethylaminocarboxamide)phenylmethyl-1,2,5,10-tetrahydropyridazino[4,5-*b*]quinoline-1,10-dione;
7-Chloro-4-hydroxy-2-(4-(*tert*-butyl)aminocarboxamide)phenylmethyl-1,2,5,10-tetrahydropyridazino[4,5-*b*]quinoline-1,10-dione;
7-Chloro-4-hydroxy-2-(4-pyrrolidinylcarboxamide)phenylmethyl-1,2,5,10-tetrahydropyridazino[4,5-*b*]quinoline-1,10-dione;

7-Chloro-4-hydroxy-2-(3-pyrrolidinylcarboxamide)phenylmethyl-1,2,5,10-tetrahydropyridazino[4,5-*b*]quinoline-1,10-dione;
7-Chloro-2-(3-aminomethylphenyl)-4-hydroxy-1,2,5,10-tetrahydropyridazino[4,5-*b*]quinoline-1,10-dione;
7-Chloro-4-hydroxy-2-(4-phenylaminocarboxamide)phenylmethyl-1,2,5,10-tetrahydropyridazino[4,5-*b*]quinoline-1,10-dione;
7-Chloro-4-hydroxy-2-(3-phenylaminocarboxamide)phenylmethyl-1,2,5,10-tetrahydropyridazino[4,5-*b*]quinoline-1,10-dione;
7-Chloro-4-hydroxy-2-(4-iodophenylmethyl)-1,2,5,10-tetrahydropyridazino[4,5-*b*]quinoline-1,10-dione;
7-Chloro-4-hydroxy-2-(4-(3-proparginol)phenyl)-1,2,5,10-tetrahydropyridazino[4,5-*b*]quinoline-1,10-dione;
{3-[(7-Chloro-4-hydroxy-1,10-dioxo(2,5-dihydropyridazino[4,5-b]quinolin-2-yl))methyl]phenyl}-N-methoxy-N-methylcarboxamide;
{3-[(7-Chloro-4-hydroxy-1,10-dioxo(2,5-dihydropyridazino[4,5-b]quinolin-2-yl))methyl]phenyl}-N-methylcarboxamide;
{3-[(7-Chloro-4-hydroxy-1,10-dioxo(2,5-dihydropyridazino[4,5-b]quinolin-2-yl))methyl]phenyl}-N-2-methoxyethyl)carboxamide;
{3-[(7-Chloro-4-hydroxy-1,10-dioxo(2,5-dihydropyridazino[4,5-b]quinolin-2-yl))methyl]phenyl}-N,N-bis(2-ethoxyethyl)carboxamide, and
7-Chloro-4-hydroxy-2-{[3-(piperazinylcarbonyl)phenyl]methyl}-1,2,5,10-tetrahydropyridazino[4,5-*b*]quinoline-1,10-dione,

or pharmaceutical ly-acceptable salts thereof.

Still more particular embodiments of the invention are those where the method comprises treatment with an exemplary compound specifically disclosed herein.

Another aspect of the invention is a method for making compounds in accord with structural diagram I.

Yet other aspects of the invention are pharmaceutical compositions which contain a compound in accord with structural diagram I; the use of compounds in accord with structural diagram I for the preparation of medicaments and pharmaceutical compositions, and a method comprising binding a compound of the invention to the NMDA receptor glycine site of a warm-blooded animal, such as a human being, so as to beneficially inhibit the activity of the NMDA receptor.

## Detailed Description of the Invention

Compounds of the invention are those within the scope of the generic description and particularly those compounds exemplified hereafter.

Suitable pharmaceutically-acceptable salts of compounds of the invention include acid addition salts such as methanesulphonate, fumarate, hydrochloride, hydrobromide, citrate, tris(hydroxymethyl)aminomethane, maleate and salts formed with phosphoric and sulphuric acid. In other embodiments, suitable salts are base salts such as an alkali metal salts for example sodium, alkaline earth metal salts for example calcium or magnesium, organic amine salts for example triethylamine, morpholine, *N*-methylpiperidine, *N*-ethylpiperidine, procaine, dibenzylamine, choline, *N,N*-dibenzylethylamine or amino acids such as lysine.

Another aspect of the invention is a process for making compounds of the invention, which process comprises the following steps:

Another aspect of the invention is a process for making compounds of the invention, which process comprises the following steps:

a) Preparing a Boc-protected hydrazine by reacting a ketone or an aldehyde, according to one of the procedures shown in the following scheme, or alternatively by reacting an alkyl halide as shown in the following scheme:

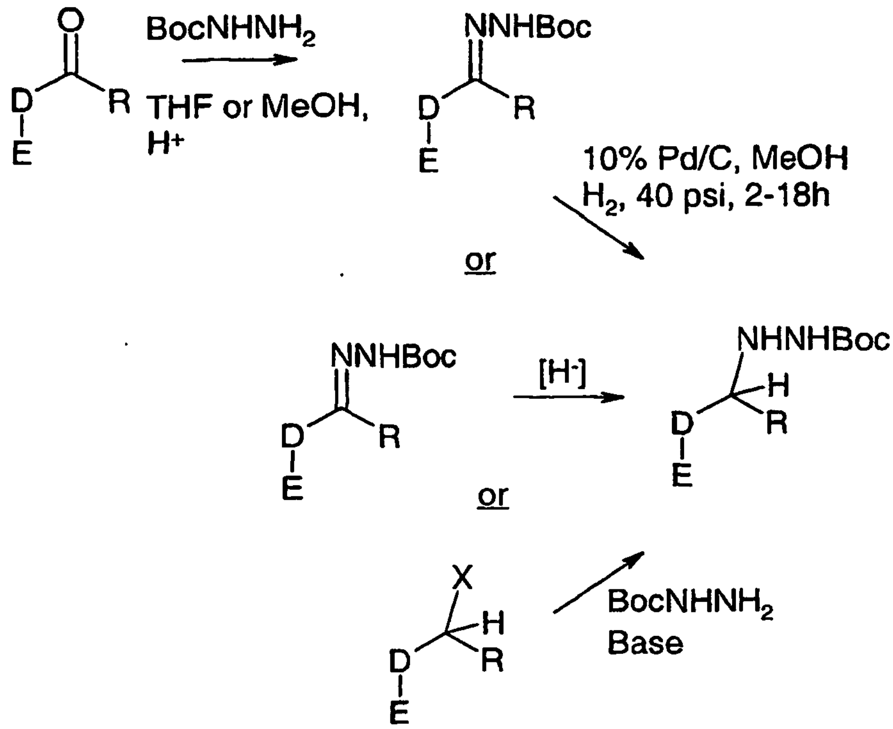


X = Cl, Br or OMs; R = H or alkyl.

Alternatively, a hydrazine may be synthesized from an aromatic aldehyde by the procedure shown in the following scheme:

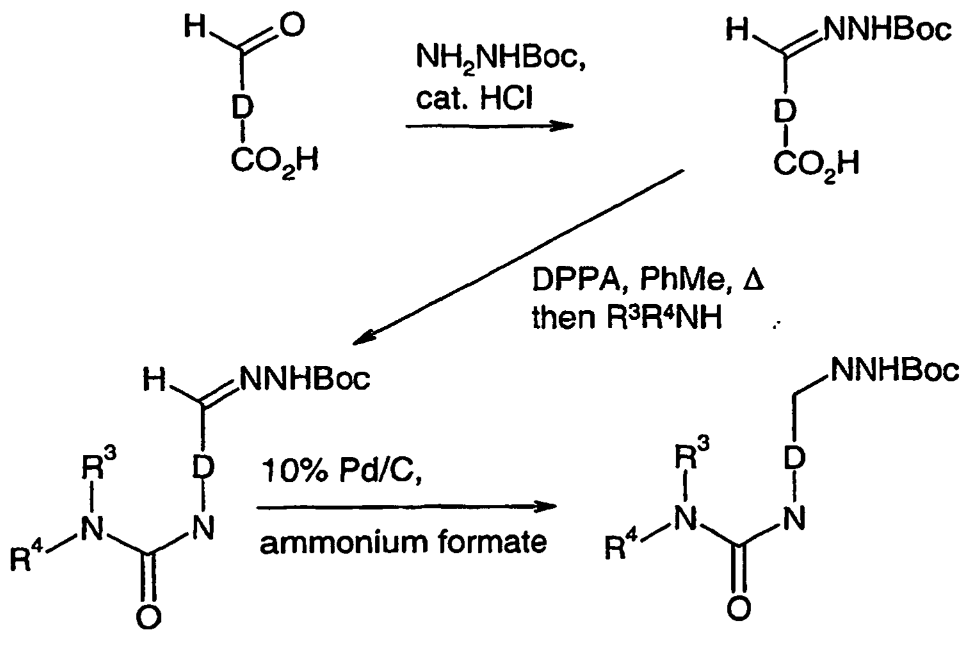


b) coupling said Boc-protected hydrazine and cyclizing the product according to the process of the following scheme to form a compound according to structural diagram I:

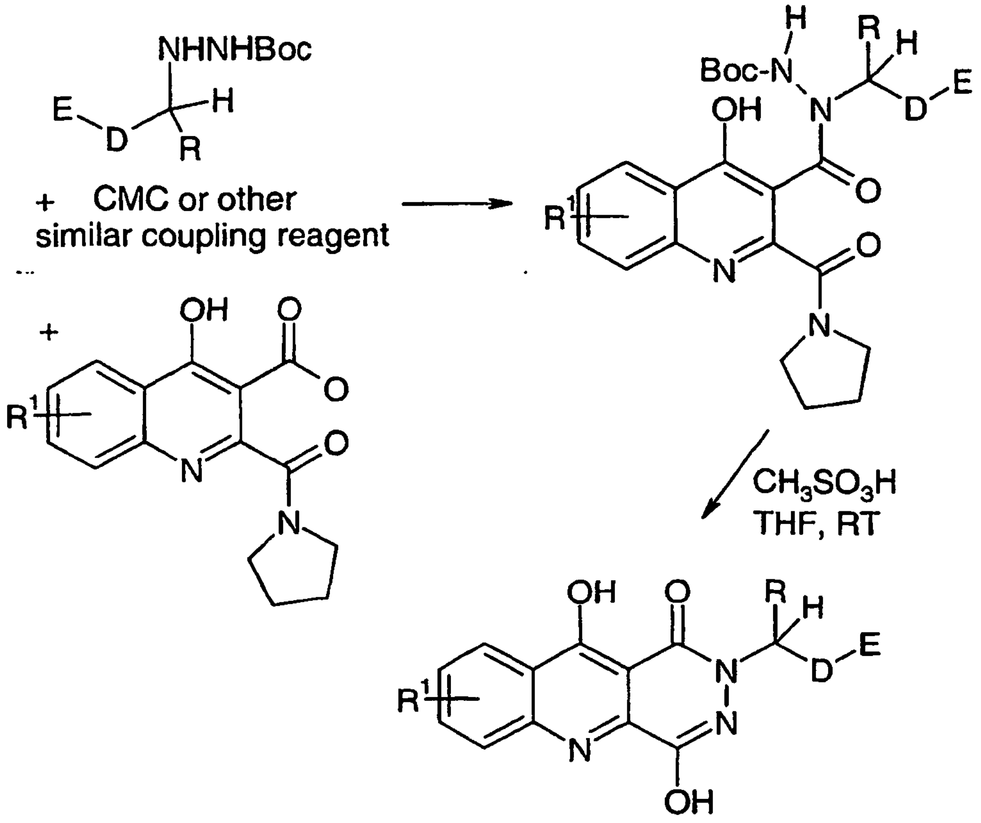


wherein:

CMC is 1-cyclohexyl-3-(2-morpholinoethyl)carbodiimide metho-p-toluenesulfonate; the "R/H/D-E" group is the "-A-D-E" moiety of structural diagram I, and throughout the foregoing process $R^1$ is as defined for structural diagram I.

Compounds within the scope of structural diagram I wherein moiety D thereof is an amide-substituted aryl may be prepared from aldehydes synthesized as follows:

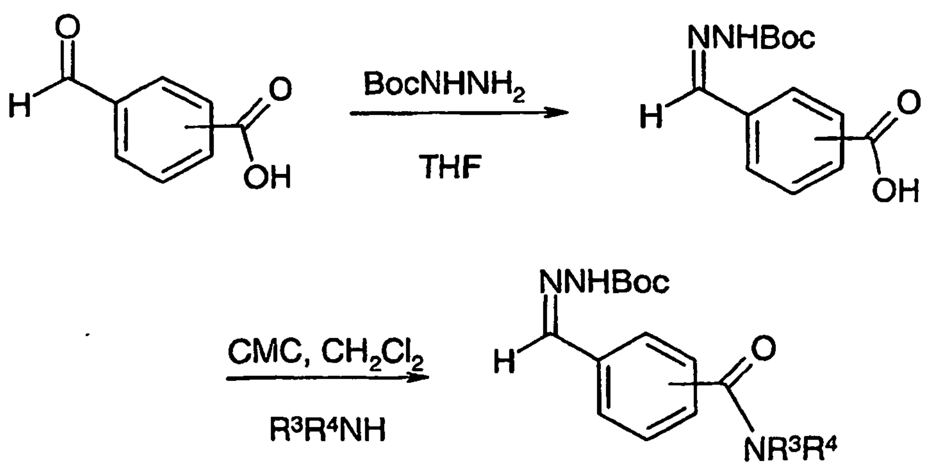


Compounds within the scope of structural diagram I wherein moiety D thereof is a urea-substituted aryl may be prepared from aldehydes synthesized as follows:

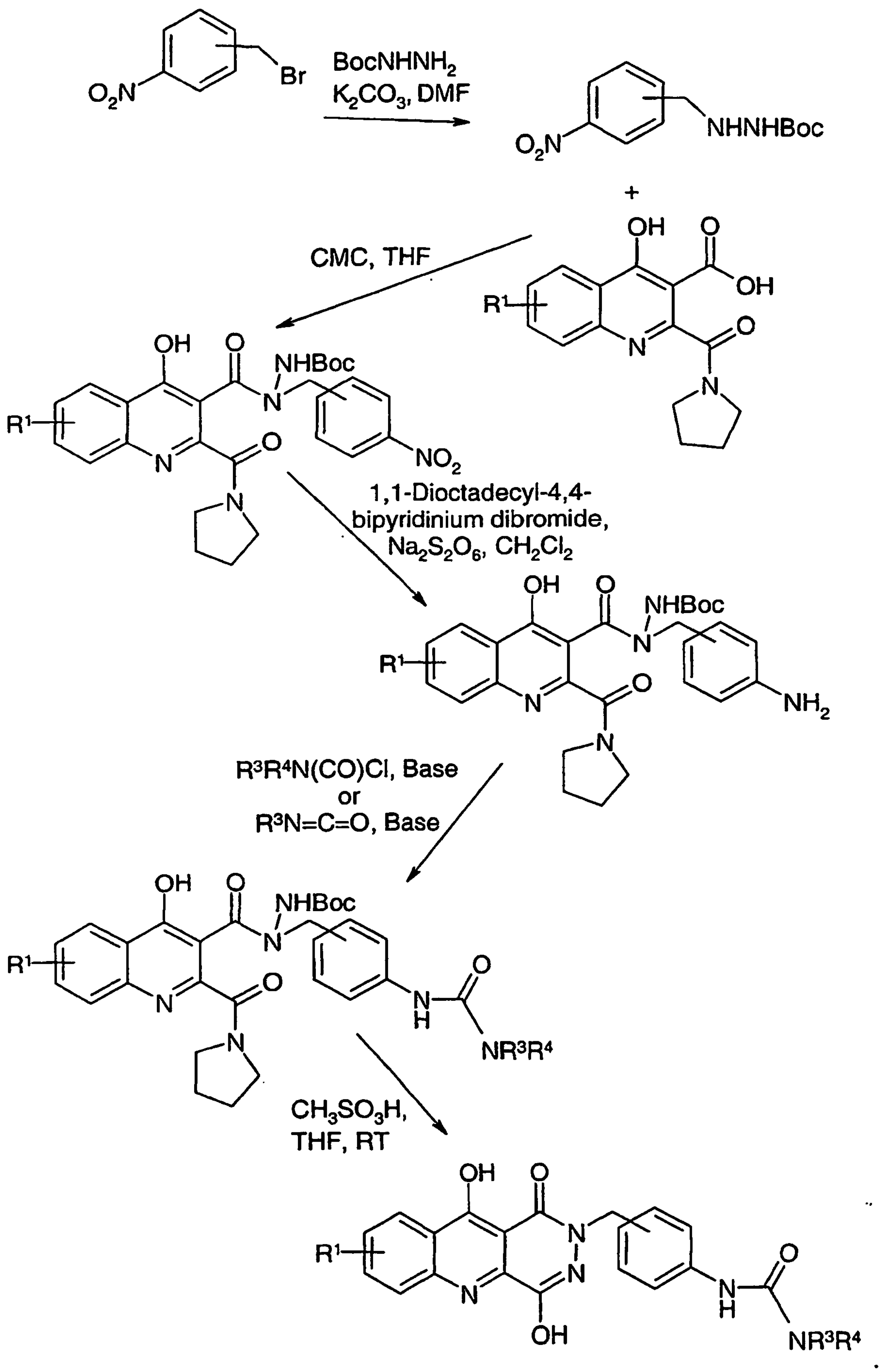
BocNHNH2
K2CO3, DMF
CMC, THF
1,1-Dioctadecyl-4,4-bipyridinium dibromide,
Na2S2O6, CH2Cl2
R3R4N(CO)Cl, Base
or
R3N=C=O, Base
CH3SO3H,
THF, RT

To use a compound of the invention or a pharmaceutically-acceptable salt thereof for the therapeutic treatment, which may include prophylactic treatment, of pain in mammals, which may be humans, the compound can be formulated in accordance with standard pharmaceutical practice as a pharmaceutical composition.

Suitable pharmaceutical compositions that contain a compound of the invention may be administered in conventional ways, for example by oral, topical, parenteral, buccal, nasal, vaginal or rectal administration or by inhalation. For these purposes a compound of the invention may be formulated by means known in the art into the form of, for example, tablets, capsules, aqueous or oily solutions, suspensions, emulsions, creams, ointments, gels, nasal sprays, suppositories, finely divided powders or aerosols for inhalation, and for parenteral use (including intravenous, intramuscular or infusion) sterile aqueous or oily solutions or suspensions or sterile emulsions. A preferred route of administration is orally by tablet or capsule.

In addition to a compound of the present invention a pharmaceutical composition of this invention may also contain one or more other pharmacologically-active agents, or such pharmaceutical composition may be simultaneously or sequentially co-administered with one or more other pharmacologically-active agents.

Pharmaceutical compositions of this invention will normally be administered so that a pain-ameliorating effective daily dose is received by the subject. The daily dose may be given in divided doses as necessary, the precise amount of the compound received and the route of administration depending on the weight, age and sex of the patient being treated and on the particular disease condition being treated according to principles known in the art. A preferred dosage regime is once daily.

A further embodiment of the invention provides a pharmaceutical composition which contains a compound of the structural diagram I as defined herein or a pharmaceutically-acceptable salt thereof, in association with a pharmaceutically-acceptable additive such as an excipient or carrier.

A yet further embodiment of the invention provide the use of a compound of the structural diagram I, or a pharmaceutically-acceptable salt thereof, in the manufacture of a medicament useful for binding to the NMDA receptor glycine site in a warm-blooded animal such as a human being.

Still another embodiment of the invention provides a method of binding a compound of the invention to the NMDA receptor glycine site of a warm-blooded animal, such as a human being, in need of treatment for pain, which method comprises administering to said animal an effective amount of a compound of structural diagram I or a pharmaceutically-acceptable salt thereof.

Definitions:

When used herein the term "alkyl" includes both straight and branched chain alkyl groups but references to individual alkyl groups such as "propyl" refer to the straight chain moiety.

When used herein the term "halo" means fluoro, chloro, bromo and iodo.

When used herein the term "aryl" means an unsaturated carbon ring or a benz-derivative thereof. Particularly, aryl means phenyl, naphthyl or biphenyl. More particularly aryl means phenyl.

When used herein the term "heteroaryl" or "heteroaryl ring" means, unless otherwise further specified, a monocyclic-, bicyclic- or tricyclic- 5-14 membered ring that is unsaturated or partially unsaturated, with up to five ring heteroatoms selected from nitrogen, oxygen and sulphur wherein a $-CH_2-$ group can optionally be replaced by a -C(O)-, and a ring nitrogen atom may be optionally oxidized to form the N-oxide. Examples of such heteroaryls include thienyl, furyl, pyranyl, pyrrolyl, imidazolyl, pyrazolyl, thiazolyl, oxazolyl, isoxazolyl, pyridyl, pyridyl-*N*-oxide, oxopyridyl, oxoquinolyl, pyrimidinyl, pyrazinyl, oxopyrazinyl, pyridazinyl, indolinyl, benzofuranyl, benzimidazolyl, benzothiazolyl, quinolyl, isoquinolinyl, quinazolinyl, xanthenyl, quinoxalinyl, indazolyl, benzofuranyl and cinnolinolyl.

When used herein the term "heterocyclyl" or "heterocyclic ring" means, unless otherwise further specified, a mono- or bicyclic- 5-14 membered ring, that is totally saturated, with up to five ring heteroatoms selected from nitrogen, oxygen and sulphur wherein a $-CH_2-$ group can optionally be replaced by a -C(O)-. Examples of such heterocyclyls include morpholinyl, pyrrolidinyl, imidazolidinyl, pyrazolidinyl, piperidinyl, piperazinyl, homopiperidinyl, homopiperazinyl and quinuclidinyl.

When used herein, where optional substituents are selected from "one or more" groups it is to be understood that this encompasses compounds where all substituents are chosen from one of the specified groups and compounds where substituents are chosen from more than one of the specified groups.

Generally in the methods, processes and examples described herein:

concentrations were carried out by rotary evaporation *in vacuo*;
operations were carried out at ambient temperature, that is in the range 18-26 °C and under a nitrogen atmosphere;
column chromatography (by the flash procedure) was performed on Merck Kieselgel silica (Art. 9385) unless otherwise stated;
yields are given for illustration only and are not necessarily the maximum attainable;

the structure of the end-products of the formula I were generally confirmed by NMR and mass spectral techniques, proton magnetic resonance spectra were determined in DMSO-$d_6$ unless otherwise stated using a Varian Gemini 2000 spectrometer operating at a field strength of 300 MHz; chemical shifts are reported in parts per million downfield from tetramethylsilane as an internal standard (δ scale) and peak multiplicities are shown thus: s, singlet; bs, broad singlet; d, doublet; AB or dd, doublet of doublets; t, triplet, dt, double of triplets, m, multiplet; bm, broad multiplet; fast-atom bombardment (FAB) mass spectral data were obtained using a Platform spectrometer (supplied by Micromass) run in electrospray and, where appropriate, either positive ion data or negative ion data were collected, in this application, $(M+H)^+$ is quoted; IR data was obtained with a Nicolet Avatar 360 FT-IR;
intermediates were not generally fully characterized and purity was in general assessed mass spectral (MS) or NMR analysis.

The following abbreviations and definitions when used, have the meanings, as follows:

| | |
|---|---|
| $CDCl_3$ | is deuterated chloroform; |
| CMC | is 1-cyclohexyl-3-(2-morpholinoethyl)carbodiimide metho-*p*-toluenesulfonate; |
| DCM | is dichloromethane; |
| DCU | is dicyclohexyl urea; |
| DHC | is 1,3-dicyclohexylcarbodiimide; |
| DMAP | is 4-(dimethylamino)pyridine; |
| DMF | is N,N-dimethylformamide; |
| DMSO | is dimethylsulphoxide; |
| m/s | is mass spectroscopy; |
| NMP | is N-methylpyrrolidone; |
| NMR | is nuclear magnetic resonance; |
| p.o. | is *per os;* |
| THF | is tetrahydrofuran, and |
| t.i.d. | is three times daily. |

The examples and tests described herein are intended to illustrate but not limit the invention.

**Examples:**

**Example 1:** 7-Chloro-4-hydroxy-2-(2,4,6-trimethylphenyl)-1,2,5,10-tetrahydropyridazino[4,5-*b*]quinoline-1,10-dione. (*tert*-Butoxy)-N-[(2,4,6-trimethylphenyl)amino]carboxamide:

A suspension of 2,4,6-trimethylphenylhydrazine hydrochloride (15.02 g, 80.46 mmol) in saturated aqueous $NaHCO_3$ was stirred for 10 minutes and then treated with solid $K_2CO_3$ (18.97 g, 137.25 mmol). The resulting fine light yellow suspension was stirred for 10 minutes. A solution of di-*tert*-butyldicarbonate (25.03 g, 89.00 mmol) in 375 mL THF was added over 5 minutes and the resulting biphasic mixture was vigorously stirred for 3 hours. The reaction mixture was partitioned in water and the aqueous layer was extracted with diethyl ether (3 x 75 mL). The combined organic layers were washed with saturated NaCl (2 x 100 mL) and distilled water (2 x 100 mL), dried over $MgSO_4$, and concentrated under reduced pressure. Drying *in vacuo* afforded an orange oil which crystallized upon standing. The material was triturated in 5% diethyl ether in hexanes overnight to afford a beige solid which was isolated by filtration (13.82 g). The solid was recrystallized from diethyl ether/hexanes to afford 11.85 g (59%) of the desired product as a cream colored solid. The filtrates from the trituration and crystallization were combined and concentrated to afford 13.50 g of material which was purified by flash chromatography on silica gel using 25:75 diethyl ether:hexanes as eluant. This afforded an additional 4.69 g (23%) of the title compound. $^1H$ NMR (300 MHz, $CDCl_3$): δ 6.79 (s, 2 H), 6.25 (bs, 1 H), 5.65 (bs, 1 H), 2.34 (s, 6 H), 2.22 (s, 3 H), 1.40 (s, 9 H); MS (CI) *m*/*z* 249.

Dimethyl 7-chloro-4-hydroxyquinoline-2,3-dicarboxylate:

A stirred mixture of methyl 2-amino-4-chlorobenzoate (2.50 g, 13.5 mmol) and dimethyl acetylenedicarboxylate (2.05 g, 14.4 mmol) in *tert*-butanol (22 ml) was refluxed for 7 hours under a nitrogen atmosphere. After adding additional dimethyl acetylenedicarboxylate (1.16 g, 8.13 mmol) and refluxing another 2.5 hours, the reaction mixture was allowed to cool to room temperature and potassium tert-butoxide (1.56 g, 13.9 mmol) was added in one portion. A precipitate formed and the resulting mixture was refluxed for 1.5 hours. The mixture was cooled to room temperature and filtered to separate the solids, which were washed with *tert*-butanol and diethyl ether. The solids were dissolved in water and acidified with 1 N sulfuric acid to form a precipitate. The resulting mixture was extracted with DCM and the combined

extracts were washed with brine and water, dried over $MgSO_4$, filtered and concentrated to give a green solid. Recrystallization of this material from methanol provided the title compound (1.15 g, 47%) as an off-white solid, mp 232-233 °C; MS (Cl):296 (M+H). Analysis for $C_{13}H_{10}ClNO_5$: Calc'd: C, 52.81; H, 3.41; N, 4.74; Found: C, 52.75; H, 3.47; N, 4.69.

3-Carbomethoxy-7-chloro-4-hydroxyquinoline-2-carboxylic acid:

To a stirred suspension of dimethyl 7-chloro-4-hydroxyquinoline-2,3-dicarboxylate (1.0 g, 3.38 mmol) in water (20 mL) was added an aqueous solution of sodium hydroxide (0.27 g, 6.75 mmol). Upon addition, the suspension dissolved. The reaction mixture was warmed to 60 °C for 1 hour. After this time the reaction was cooled to room temperature and acidified with concentrated hydrochloric acid. The product was then extracted into diethyl ether and ethyl acetate. The organic extracts were dried over $MgSO_4$, filtered and concentrated in *vacuo* to provide the title compound as a solid (900 mg). This material was purified by recrystallization employing an ethyl acetate/hexane co-solvent system to provide the title compound (571 mg, 60%) as a white solid mp 296 °C (dec); MS (CI) = 238 (M+H). Analysis for $C_{12}H_8NO_5Cl\bullet 0.45\ CH_3CO_2CH_2CH_3\bullet 0.10\ H_2O$: Calc'd: C, 51.30; H, 3.68; N 4.34, Found: C, 51.28; H, 3.62; N 3.97 $^1$H NMR 8.22 (d, J = 8.7 Hz, 1H), 7.92 (d, J = 1.8 Hz, 1H), 7.28 (dd, J = 8.7, 1.8 Hz, 1H), 3.90 (s, 3H).

3-Carbomethoxy-2-pyrrolidinocarbamide-7-chloro-4-hydroxyauinoline:

To a suspension of 3-carbomethoxy-7-chloro-4-hydroxyquinoline-2-carboxylic acid (2.25 g, 8.0 mmol) in THF (20 mL) at ambient temperature under a $N_2$ atmosphere was added DHC (1.65 g, 8.0 mmol) and pyrrolidine (0.596 g, 8.4 mmol). The reaction was stirred room temperature for 15 hours after which time the by-product urea was removed via filtration. The desired product was purified via flash column chromatography employing 5% methanol in chloroform to provide the title compound (2.52 g, 94.3%) as a tan solid, mp = 215°C; MS (CI): 335 (M+H). 300 MHz $^1$H NMR (DMSO-$d_6$): δ 8.12 (d, J = 8.7 Hz, 1H), 7.60 (d, 1H, J =1.8 Hz), 7.47 (dd, 1H, J = 8.8, 2.0 Hz), 3.69 (s, 3H), 3.40-3.49 (m, 2H), 3.27-3.33 (m, 2H), 1.80-1.96 (m, 4H).

7-Chloro-4-oxo-2-(pyrrolidinylcarbonyl)hydroquinoline-3-carboxylic acid:

To a suspension of 3-carbomethoxy-2-pyrrolidinocarbamide-7-chloro-4-hydroxy quinoline (2.52g, 7.5 mmol) in de-ionized water (40 mL) was added dropwise a solution (20 mL) of an aqueous potassium hydroxide (882 mg, 15.75 mmol). Upon complete addition, the reaction was warmed to 60 °C. After 3 hours, the reaction was filtered to remove a small amount of insoluble material. The filtrate was then acidified to pH = 1 which yield a white precipitate. The solid was isolated by vacuum filtration, washed with water, and dried at 30 °C *in vacuo* for 16 hours. This provided the title compound (1.5 g, 64%) as a white solid, mp = 225-8 °C; MS (CI): 321 (M+H). 300 MHz $^1$H NMR (DMSO-$d_6$): δ 8.28 (d, J = 8.8 Hz, 1H), 7.77 (s, 1H), 7.64 (d, 1H, J = 8.7), 3.52-3.57 (m, 2H), 3.17-3.19 (m, 2H), 1.83-1.98 (m, 4H).

N-[(*tert*-Butoxy)carbonylamino][7-chloro-4-oxo-2-(pyrrolidinylcarbonyl)(3-hydroquinolyl)]-N-(2,4,6-trimethylphenyl) carboxamide.

To a stirred suspension of 7-chloro-4-oxo-2-(pyrrolidinylcarbonyl)hydroquinoline-3-carboxylic acid (15.25 g, 47.55 mmol) in anhydrous THF (730 mL) under nitrogen was added DHC (11.66 g, 56.51 mmol). The (*tert*-butoxy) -N-[(2,4,6-trimethyl phenyl)amino]carboxamide (16.54 g, 66.07 mmol) was dissolved in 270 mL anhydrous THF and added to the bright yellow reaction mixture. The reaction mixture was stirred at room temperature for 5 hours and then heated at 60 °C for 20 hours. After cooling to room temperature, the mixture was filtered and the solid material (DCU) was washed with diethyl ether. The filtrate was concentrated and then dried *in vacuo* to afford a brown oil. The oil was purified by flash chromatography on silica gel using a gradient of 99:1 to 95:5 DCM:methanol as eluant. Concentration of the appropriate fractions provided a tan solid (21.40 g) which was sonicated in methanol and filtered to remove the remaining DCU from the desired product. This purification procedure was repeated until all DCU was removed. A total of 18.04 g (69%) of the title compound was obtained. $^1$H NMR (300 MHz, DMSO, $d_6$): δ 13.14 (s, 1 H), 9.08 (bs, 1 H), 8.28 (d, 1 H, $J_o$=8.7 Hz), 7.69 (d, 1 H, $J_m$=1.6 Hz), 7.56 (dd, 1 H, $J_o$=8.7 Hz, $J_m$=1.6 Hz), 6.88 (s, 2 H), 3.44 (m, 4 H), 2.32 (s, 6 H), 2.24 (s, 3 H), 1.86 (m, 4 H), 1.09 (bs, 9 H); MS (-CI) *m*/*z* 552.

7-Chloro-4-hydroxy-2-(2,4,6-trimethylphenyl)-1,2,5,10-tetrahydropyridazino[4,5-*b*]quinoline-1,10-dione.

To a stirred suspension of N-[(*tert*-butoxy)carbonylamino][7-chloro-4-oxo-2-(pyrrolidinylcarbonyl)(3-hydroquinolyl)]-N-(2,4,6-trimethyl phenyl)carboxamide (3.72 g, 6.65 mmol) in 180 mL anhydrous THF under nitrogen was added methanesulfonic acid (21.0 mL, 32.40 mmol). The resulting bright yellow solution was stirred at room temperature for 18 hours. The solution was poured into 1.7 L water, stirred 1 hour, and filtered to afford a yellow solid. After sonication

in methanol, the title compound was obtained by filtration as a beige solid (1.21g, 47%), m.p. 187-189°C. $^1$H NMR (300 MHz, DMSO, $d_6$): δ 12.79 (bs, 1 H), 12.06 (bs, 1 H), 8.16 (d, 1 H, $J_o$=8.7 Hz), 8.07 (d, 1 H, $J_m$=1.3 Hz), 7.46 (dd, 1 H, $J_o$=8.7 Hz, $J_m$=1.3 Hz), 2.29 (s, 3 H), 2.02 (s, 6 H); MS (CI) *m*/*z* 382/384. Calc'd. for $C_{20}H_{16}ClN_3O_3$•0.3 $H_2O$: C, 62.04; H, 4.32; N, 10.85. Found C, 62.01; H, 4.22; N, 10.71.

**Example 2:** 7-Chloro-4-hydroxy-2-(4-carbomethoxy-phenylmethyl)-1,2,5,10-tetrahydropyridazino[4,5-*b*]quinolin-1,10-dione. Methyl 4-({[(*tert*-butoxy)carbonylamino]amino}methyl)benzoate.

A solution of methyl 4-bromomethylbenzoate (25.0 g, 0.11 moles) and *tert*-butylcarbazate (68.65 g, 0.52 moles) in DMF (170 mL) was stirred and heated to 50°C under nitrogen. Triethylamine (21.1 g, 0.21 moles) was added and the reaction mixture was heated at 50°C for an additional 10 minutes. The reaction mixture was poured into water (1 L) and the resulting mixture extracted with DCM (4 x 350 mL). The combined organic layers were washed with water (4 x 200 mL) and saline (1 x 300 mL) and then dried ($MgSO_4$). Concentration of the filtered organic layer *in vacuo* provided a yellow oil. This material was purified by silica gel column chromatography using diethyl ether/hexane (1/1) as the eluant to obtain the title compound as a clear colorless oil (26.50 g, 87% yield). $^1$H NMR (300 MHz, DMSO-$d_6$): δ 1.38 (s, 9H); 3, 84 (s, 3H,); 3.92 (d, 2H, J = 4.2 Hz); 4.95 (d, 1H, J = 4.2 Hz); 7.47(d, 2H, J = 8.4 Hz); 7.90 (d, 2H, J = 8.1 Hz); 8.29 (s, 1H).

N-[(*tert*-butoxy)carbonylamino][7-chloro-4-oxo-2-(pyrrolidinylcarbonyl)(3-hydroquinolyl)]-N-(4-carbomethoxybenzyl) carboxamide.

To a stirred mixture of 7-chloro-4-oxo-2-(pyrrolidinylcarbonyl)hydroquinoline-3-carboxylic acid, Example 1, (18.38 g, 57 mmol) and dry THF (800 mL) under nitrogen was added DHC (15.25 g, 74 mmol) in one portion. After stirring the reaction mixture for 5 minutes, a solution of methyl 4-({[(*tert*-butoxy)-carbonylamino]amino}methyl)benzoate (24.1 g, 86 mmol) and THF (200 mL) was rapidly added and the mixture was stirred overnight at room temperature. The reaction mixture was concentrated under reduced pressure to a solid-oil mixture. Using methanol the material was preabsorbed onto silica gel. Silica gel column chromatography using methanol/chloroform (5/95) as eluant gave the title compound as an tan solid (33.43g, 100 %) containing DCU. This material was used in the subsequent reaction.

7-Chloro-4-hydroxy-2-(4-carbomethoxy-phenylmethyl)-1,2,5,10-tetrahydropyridazino[4,5-*b*]quinolin-1,10-dione.

To a stirred mixture of N-[(*tert*-butoxy)carbonylamino][7-chloro-4-oxo-2-(pyrrolidinylcarbonyl)(3-hydroquinolyl)] -N-(4-carbomethoxybenzyl)carboxamide (33.6 g, 57.3 mmol) and dry THF (1156 mL) under nitrogen was added methanesulfonic acid (140 mL, 207.3 g, 2.16 moles) all at once. The mixture was stirred overnight at room temperature. The reaction mixture was poured into ice water (3 L) and the resulting solid filtered. The collected solids were suspended in methanol (300 mL), sonicated for 30 minutes and filtered. The collected solids were submitted to the identical sonication process two more times and the collected solids were air dried to give the title compound as a white solid (13.38 g, 40 % yield), m.p. > 290 °C. MS:) 412 (M+1, small parent ion), 410 (M-1, large parent ion) $^1$H NMR (300 MHz, DMSO-$d_6$): δ 3.83 (s, 3H), 5.18 (s, 2H); 7.43 (m, 3H,); 7.92 (d, 2H, J = 8.1 Hz); 8.02 (S, 1H); 8.14 (d, 1H, J = 8.4 Hz). Calc'd. for $C_{20}H_{14}ClN_3O_5$: C, 58.27; H, 3.56; N, 10.00 Found: C, 58.21; H, 3.51; N, 10.00.

**Example 3:** 7-Chloro-4-hydroxy-2-(4-(furan-2-ylmethyl)aminocarbonyl)-benzyl-1,2,5,10-tetrahydropyridazino[4,5-*b*] quinoline-1,10-dione.

7-Chloro-4-hydroxy-2-(4-carboxy-methylphenyl)-1,2,5,10-tetrahydropyridazino[4,5-*b*]quinoline-1,10-dione.

To a stirred suspension of 7-chloro-4-hydroxy-2-(4-carbomethoxy-methylphenyl)-1,2,5,10-tetrahydropyridazino[4,5-*b*]quinoline-1,10-dione, Example 2, (4.24 g, 10.2 mmol) in water (500 mL) at room temperature sodium hydroxide was added (1.22 g, 30.5 mmol). The slurry was stirred at 50 °C for 1.5 hours then the reaction acidified to pH ~ 1 with aqueous hydrochloric acid (12 N). The slurry was filtered and the solid dried *in vacuo* at 55°C. Analysis by $^1$H NMR showed the solid to be a mixture of starting ester and desired product of the ratio 33/66% respectively. The reaction material was resubjected to the above conditions for 18 hours then acidified to pH ~ 1 with aqueous hydrochloric acid (12 N). The slurry was filtered to give the title compound as a tan solid (1.60 g, 39%). $^1$H NMR (300 MHz, DMSO-$d_6$): δ 5.18 (s, 2H); 7.46 (m, 3H); 7.92 (d, 1H, J= 8.1 Hz); 8.03 (d, 1H, J = 1.8 Hz); 8.15 (d, 1H, J = 8.7 Hz)

7-Chloro-4-hydroxy-2-(4-(furan-2-ylmethyl)aminocarbonyl)-benzyl-1,2,5,10-tetrahydropyridazino[4,5-*b*]quinoline-1,10-dione.

To a stirred suspension of PS-carbodiimide (77 mg, 0.75 mmol, 0.98 mmol/g, Argonaut Technologies Inc., lot

no. 113-78) in DMF (3 mL) at room temperature was added 7-chloro-4-hydroxy-2-(4-carboxy-phenyl)-1,2,5,10-tetrahydropyridazino[4,5-*b*]quinoline-1,10-dione (100 mg, 0.25 mmol). The reaction was stirred at 50 °C for 4 hours. The slurry was filtered and the resin washed with DMF (4 x 10 mL). The resin was added to DMF (3 mL) treated with furfurylamine (97 mg, 1.0 mmol) and heated at 50 °C for 1 hour. The reaction was filtered, washed with DMF (2 x 3 mL), and the solvents were evaporated to dryness. The residual oil was treated with aqueous hydrochloric acid (1 mL, 1 N) to give the title compound as a tan solid (5 mg, 4%). MS (-CI) *m*/*z* 474 $^1$H NMR (300 MHz, DMSO-$d_6$): δ 4.45 (d, 2H, J = 5.4 Hz); 5.16 (s, 2H); 6.26 (d, 1H, J = 3 Hz); 6.39 (dd, 1H, J = 1.8, 3.0 Hz); 7.37 (d, 2H, J = 8.1 Hz); 7.45 (dd, 1H, J = 7.2, 9.0 Hz); 7.57 (s, 1H); 7.84 (d, 2H, J = 8.4 Hz); 8.03 (d, 1H, J = 1.8 Hz); 8.14 (d, 1H, J = 8.7 Hz); 8.94 (m, 1H); 11.95 (br s, 1H); 12.66 (br s,1H).

**Example 4:** 7-Chloro-4-hydroxy-2-(4-dimethylaminobenzyl)-1,2,5,10-tetrahydropyridazino[4,5-*b*]quinoline-1,10-dione methanesulfonate. (*tert*-Butoxy)-N-[(4-dimethylaminobenzyl)amino]carboxamide.

To a stirred solution of *tert*-butylcarbazate (3.14 g, 23.8 mmol) in ethanol (40 mL) at reflux was added 4-dimethylaminobenzaldehyde (2.50 g, 16.8 mmol) over 5 minutes and the mixture stirred at reflux for 2 days. The reaction mixture was cooled to room temperature, then added to 5% palladium-on-carbon (425 mg) and ethanol (20 mL) in a Parr shaker bottle. The reaction was hydrogenated at 50 psi for 24 h. The mixture was filtered through diatomaceous earth, which was washed with methyl alcohol. The combined filtrate and washes were concentrated *in vacuo.* The material was subjected to flash chromatography (silica gel, 25% ethyl acetate/toluene) to yield the title compound as an oil (3.5 g, 78%). $^1$H NMR (300 MHz, DMSO-$d_6$): δ 1.39 (s, 9H); 2.86 (s, 6H); 3.71 (d, 2H, J = 5.1 Hz); 4.40 (dt, 1H, J = 4.8, 5.1 Hz); 6.67 (d, 2H, J = 8.7 Hz); 7.11 (d, 2H, J = 8.7 Hz); 8.18 (s, 1H, br).

N-[(*tert*-butoxy)carbonylamino][7-chloro-4-oxo-2-(pyrrolidinylcarbonyl)(3-hydroquinolyl)]-N-(4-dimethylaminobenzyl) carboxamide.

To a stirred mixture of 7-chloro-4-oxo-2-(pyrrolidinylcarbonyl)hydroquinoline-3-carboxylic acid, Example 1, (2.8 g, 8.7 mmol) and dry THF (130 mL) under nitrogen was added CMC (5.6 g, 13.1 mmol). The yellow reaction mixture was treated rapidly with (*tert*-butoxy)-N-[(4-dimethylaminobenzyl)amino]carboxamide (1.6 g, 7.1 mmol) in THF (40 mL). After stirring overnight, the reaction mixture was filtered, and the filtrate was concentrated to give a crusty yellow foam (6.85 g). The material was subjected to flash chromatography (silica gel, 5% MeOH/DCM) to give the title compound (4.11 g, 83%).

7-Chloro-4-hydroxy-2-(4-dimethylaminobenzyl)-1,2,5,10-tetrahydropyridazino[4,5-*b*]quinoline-1,10-dione methanesulfonate.

To a stirred mixture of N-[(*tert*-butoxy)carbonylamino][7-chloro-4-oxo-2-(pyrrolidinylcarbonyl)(3-hydroquinolyl)] -N-(4-dimethylaminobenzyl)carboxamide (3.5 g, 6.2 mmol) and dry THF (120 mL) under nitrogen was added methanesulfonic acid (15.3 mL, 0.23 mol). The clear yellow solution was stirred for 2 days at room temperature. The precipitate that formed was collected by suction filtration and washed with THF and diethyl ether. The solid was treated with methanol and sonicated for 30 minutes. The solid was again collected by filtration, washed with methanol and diethyl ether, and dried at 90 °C *in vacuo* to give the title compound (2.3 g, 72%) as a white powder, m.p. softens 218-225 °C, decomposes 270-280 °C. $^1$H NMR (300 MHz, DMSO-$d_6$, TFA shake): δ 2.54 ($CH_3CO_2H$, s, 3H)3.23 (s, 6H), 5.18 (s, 2H); 7.44 (dd, 1H, J = 1.8, 8.7 Hz); 7.51 (d, 2H, J = 8.4 Hz); 7.67 (d, 2H, J = 8.4 Hz); 8.08 (d, 1H, J = 1.8 Hz); 8.19 (d, 1 H, J = 8.7 Hz). Calc'd. for $C_{20}H_{17}ClN_4O_3$●$CH_3SO_3H$●$H_2O$: C, 49.36; H, 4.54; N, 10.96. Found: C, 49.22; H, 4.51; N, 10.93.

**Example 5:** 7-Chloro-4-hydroxy-2-(benzimidazol-5-ylmethyl)-1,2,5,10-tetrahydropyridazino[4,5-*b*]quinoline-1,10-dione methanesulfonate. N-((1-Aza-2-benzimidazole-5-yl)vinyl)(*tert*-butoxy) carboxamide.

To a stirred solution of *tert*-butylcarbazate (2.49 g, 18.9 mmol) in THF (40 mL) was added benzimidazole-5-carboxaldehyde (2.77 g, 18.9 mmol) in THF (20 mL). The reaction was stirred overnight and the solvent evaporated to give the title compound as a white solid (3.88 g, 79%). $^1$H NMR (300 MHz, DMSO-$d_6$): δ 1.48 (s, 9H); 7.57 (d, 1H, J = 7.2 Hz); 7.62 (d, 1H, J =7.2 Hz); 7.79 (s, 1H); 8.12 (s, 1H); 8.23 (s, 1H); 10.83 (s, 1H)

(*tert*-Butoxy)-N-[(benzimidazol-5-ylmethyl)]carboxamide.

N-((1-Aza-2-benzimidazole-5-yl)vinyl)(*tert*-butoxy) carboxamide (0.500 g, was dissolved in ethyl alcohol (80 mL) and placed in a Parr shaker bottle. Hydrochloric acid (1.92 mL of 1M solution in ethyl alcohol) was added, followed by 10% palladium-on-carbon (750 mg). The reaction was hydrogenated at 40 psi for 2.5 hr and then filtered through

diatomaceous earth to remove the catalyst. The catalyst/diatomaceous earth was washed with ethyl alcohol (3 x 100 mL) and the combined filtrate and washes were concentrated *in vacuo*. The residual oil (ca. 0.54 g) was dissolved in water (3 mL) to which a small portion of saturated sodium bicarbonate was then added (ca. 2 mL) followed by saturated sodium chloride (ca. 2 mL). The resulting aqueous layer was then extracted with ethyl acetate (200 mL). The organic extract was dried over $MgSO_4$, filtered and evaporated to dryness to give the title compound as a white solid (0.27 g, 47%). $^1H$ NMR (300 MHz, DMSO-$d_6$): δ (TFA shake) 1.36 (s, 9H); 4.51 (s, 2H); 7.68 (dd, 1H, J =1.5, 8.5 Hz); 7.95 (d, 1H, J = 8.5 Hz); 8.00 (s, 1H); 9.68 (s, 1H).

N-[(*tert*-Butoxy)carbonylamino][7-chloro-4-oxo-2-(pyrrolidinylcarbonyl)(3-hydroguinolyl]-N-(benzimidazol-5-ylmethyl) carboxamide.

To a stirred slurry of 7-chloro-4-oxo-2-(pyrrolidinylcarbonyl)hydroquinoline-3-carboxylic acid, Example 1, (0.95 g, 3.00 mmol) in THF (40 mL) was added CMC (1.88 g, 4.50 mmol) and the reaction was stirred for five minutes. To this mixture was added dropwise a solution of (*tert*-butoxy)-N-[(benzimidazol-5-ylmethyl)]carboxamide (0.77 g, 3.00 mmol) and DMAP (ca. 0.020 g) in THF (20 mL). The mixture was stirred overnight at room temperature and then filtered. The collected solids were washed with DCM (2 x 150 mL). The combined filtrate and washes were washed with water (100 mL) and brine and then dried over $MgSO_4$, filtered and concentrated. The residual yellow foam was subjected to chromatography (silica gel, gradient of chloroform/methyl alcohol beginning from 95/5 and ending at 90/10 respectively) to give the title compound as a yellow foam (0.300 g, 19%).

7-Chloro-4-hydroxy-2-(benzimidazol-5-ylmethyl)-1,2,5,10-tetrahydropyridazino[4,5-*b*]quinoline-1,10-dione methanesulfonate.

To a stirred solution of N-[(*tert*-butoxy)carbonylamino][7-chloro-4-oxo-2-(pyrrolidinylcarbonyl)(3-hydroquinolyl] -N-(benzimidazol-5-ylmethyl) carboxamide (0.300 g, 0.53 mmol) in THF (15 mL) was added methanesulfonic acid (1.4 mL) and the reaction was stirred overnight. The reaction mixture was filtered to separate a yellow solid. This material was washed with THF and the resultant solid dried *in vacuo*. The material was then sonicated in 5 mL of 1/1 diethyl ether/methyl alcohol for fifteen minutes, filtered and washed with the same solvent system (40 mL) to give the title compound as a yellow solid (0.07 g, 25%; m.p. > 280 °C). $^1H$ NMR (300 MHz, DMSO-$d_6$): δ 2.32 ($CH_3SO_3H$); 5.29 (s, 2H); 7.45 (dd, 1H, J = 1.8, 8.7 Hz); 7.56 (d, 1H, J = 7.2 Hz); 7.81 (m, 2H); 8.03 (d, 1H, J = 2.0 Hz); 8.16 (d, 1H, J = 8.7 Hz); 9.53 (s, 1H); 12.00 (s, 1H); 12.75 (s, 1H).

**Example 6:** 7-Chloro-4-hydroxy-2-(4-hydroxy-3-methyl-benzyl)-1,2,5,10-tetrahydropyridazino[4,5-*b*]quinoline-1,10-dione. N-[1-Aza-2-(4-hydroxy-3-methylphenyl)vinyl](*tert*-butoxy)carboxamide

4-Hydroxy-3-methylbenzaldehyde (2.0 g, 14.6 mmol) was added to THF (60 mL) and to this was added *tert*-butylcarbazate (2.03 g, 15.4 mmol). The reaction mixture was stirred for 16 hours and then the solvent was removed under reduced pressure to give the title compound as a white solid (3.5 g, 95%). This material was used in the next reaction without further purification.

(*tert*-Butoxy)-N-{[(4-hydroxy-3-methylphenyl)methyl]amino}carboxamide

N-[1-Aza-2-(4-hydroxy-3-methylphenyl)vinyl](*tert*-butoxy)carboxamide (2.0 g, 7.92 mmol) was added to a Parr shaker bottle and dissolved in methyl alcohol (30 mL). To this was added 10% palladium-on-carbon (~300 mg) and the reaction was hydrogenated at 40 psi for 3 hours. The catalyst was removed by filtration through diatomaceous earth, washed with methyl alcohol (2 x 100 mL), and the solvents were removed *in vacuo* to yield an oil. The oil was dissolved in DCM, and the solvent removed *in vacuo* to give the title compound as an oil (1.89 g, 95%). $^1H$ NMR (300 MHz, DMSO-$d_6$): δ 1.38 (s, 9H); 2.08 (s, 3H); 3.62 (s, 2H); 4.40 (br s, 1H); 6.65 (d, 1H, J = 8.1 Hz); 6.88 (dd, 1H, J = 1.8, 8.1 Hz); 6.98 (s, 1H); 8.17 (br s, 1H); 9.11 (s, 1H).

N-[(*tert*-Butoxy)carbonylamino][7-chloro-4-oxo-2-(pyrrolidinylcarbonyl)(3-hydroquinolyl)]-N-[(4-hydroxy-3-methylphenyl)methyl]carboxamide

To a stirred slurry of 7-chloro-4-oxo-2-(pyrrolidinylcarbonyl)hydroquinoline-3-carboxylic acid, Example 1, (1.89 g, 5.91 mmol) in THF (50mL) was added CMC (2.99 g, 7.09 mmol) and the reaction was stirred for five minutes. To this mixture was added, via dropwise addition, a solution of (*tert*-butoxy)-N-{[(4-hydroxy-3-methylphenyl)methyl]amino}carboxamide (1.61 g, 6.38 mmol) and DMAP (0.200 g, 1.63 mmol) in THF (5 mL), and the mixture was then refluxed for 8 hours. The solution was filtered and the insolubles washed with THF (2 x 20 mL). The THF was evaporated and the

residual solid chromatographed ($SiO_2$, 95/5 chloroform/diethyl ether) to give the title compound as a yellow foam (2.1 g, 65%). This material was used in the next reaction without further purification.

7-Chloro-4-hydroxy-2-(4-hydroxy-3-methyl-benzyl)-1,2,5,10-tetrahydropyridazino[4,5-*b*]quinoline-1,10-dione.

To a stirred solution of N-[(*tert*-butoxy)carbonylamino][7-chloro-4-oxo-2-(pyrrolidinylcarbonyl)(3-hydroquinolyl)]-N-[(4-hydroxy-3-methylphenyl)methyl]carboxamide (0.30 g, 0.54 mmol) in THF (10 mL) was added methanesulfonic acid (1.2 mL) and the reaction was stirred at room temperature for 16 hours. The THF was removed under reduced pressure to yield an orange oil. To this was added diethyl ether (50 mL) and the reaction stirred for 10 minutes. The ether was carefully decanted to leave a thick orange oil. To this was added water (5 mL) and a fluffy yellow precipitate formed. The mixture was stirred for 10 minutes and then filtered. The solids were washed with water (10 mL) followed by diethyl ether (20 mL). The solids were collected and sonicated in methyl alcohol/diethyl ether (1:10, 5 mL) for 10 minutes. The solids were collected and dried under reduced pressure to give the title compound as a cream-colored solid (0.06 g, 30%). $^1$H NMR (300 MHz, DMSO-$d_6$): δ 2.07 (s, 3H); 4.94 (s, 2H); 6.69 (d, 1H, J = 8.1 Hz); 6.96 (d, 1H, J = 8.1 Hz); 7.03 (s, 1H); 7.42 (d, 1H, J = 8.4 Hz); 8.01 (s, 1H); 8.14 (d, 1H, J = 8.4 Hz); 9.23 (s, 1H); 11. 89 (s, 1H); 12.54 (s, 1H). MS (-CI): *m*/*z* 382/383.

**Example 7:** 7-Chloro-4,10-dihydroxy-2-[(2-methylthio)phenylmethyl]-2-hydropyridazino[4,5-*b*]quinoline-1-one.
2-(Methylthio)benzaldehyde.

To a stirred solution of N,O-dimethylhydroxylamine hydrochloride (3.02 g, 30.96 mmol), 1-(3-(dimethylamino) propyl)-3-ethylcarbodiimide hydrochloride (8.94 g; 51.33 mmol) and triethylamine (6.5 mL, 89.93 mmol) in DCM (250 mL) under nitrogen, was added 2-(methylthio)benzoic acid (5 g, 29.72 mmol). The reaction mixture was stirred for 2 days at room temperature under nitrogen and then 200 mL DCM was added. The reaction mixture was washed successively with water (2 x 100 mL), HCl 2 N solution (2 x 200 mL) and a saturated bicarbonate solution (200 mL), then dried over $MgSO_4$ and concentrated *in vacuo* to give N-methoxy-N-methyl(2-methylthiophenyl)carboxamide (5.16 g, 82 % yield). $^1$H NMR (300 MHz, DMSO-$d_6$); δ 2.45 (s, 3H); δ 3.19 (s, 3H); δ 3.50 (s, 3H); 7.18-7.30 (m, 2H); 7.40 (d, 2H, J = 4 Hz).

To a solution of N-methoxy-N-methyl(2-methylthiophenyl)carboxamide, prepared above, (5.16 g, 24.42 mmol) in dry THF (150 mL) at -78 °C and under nitrogen was added $LiAlH_4$ (0.93 g, 24.5 mmol) and the reaction was stirred at -78 °C for 3 hours and then allowed to warm at room temperature. Sodium sulfate decahydrate was added in excess to the reaction mixture which had been diluted with an additional amount of THF (100 mL). The solution was stirred at room temperature for 1 more hour. The reaction mixture was filtered through diatomaceous earth and the filter cake was washed with THF. The combined organic filtrate and washes were dried over $MgSO_4$, filtered and concentrated *in vacuo* to give a yellow oil (3.63 g). This oil was dissolved in ethyl acetate (100 mL) and washed with 1 N HCl (100 mL) and then with saturated sodium bicarbonate solution (100 mL). Concentration of the organic material provided the title compound (2.83g; 76%) as a yellow oil. $^1$H NMR (300 MHz, DMSO-$d_6$); δ 2.48 (s, 3H); 7.37 (t, 1H, *J* = 8 Hz); 7.45 (d, 1H, *J* = 8 Hz); 7.65 (t, 1H, *J*= 8 Hz); 7.92 (d, 1H, *J*= 8 Hz); 11.03 (s, 1H).

N-[1-Aza-2-(2-methylthiophenyl)vinyl](*tert*-butoxy)carboxamide.

To stirred solution under nitrogen and at room temperature of 2-(methylthio)benzaldehyde (2.83 g, 18.59 mmol) in dry methanol (200 mL) with acetic acid (4 mL) to adjust the pH to 3 was added *tert*-butylcarbazate (2.50 g, 18.92 mmol). The reaction mixture was stirred at room temperature for 1 hour and 2 more mL acetic acid were added to further adjust the pH to 3. After stirring the reaction mixture at room temperature overnight, the methanol was evaporated *in vacuo* and chloroform (200 mL) was added and then evaporated *in vacuo* to remove the acetic acid. The residue provided the title compound (6.32 g) as yellow oil which was used in the following step without any further purification. $^1$H NMR (300 MHz, DMSO-$d_6$); δ 1.47 (s, 9H); 2.47 (s, 3H); 7.22 (m, 1H); 7.32-7.38 (m, 2H); 7.74 (d, 1H, *J*= 8 Hz); 8.44 (s,1H); 11.03 (b, NH).

(*tert*-Butoxy)-N-{[(2-methylthiophenyl)methyl]amino}carboxamide.

To a stirred mixture of N-[1-aza-2-(2-methylthiophenyl)vinyl](*tert*-butoxy)carboxamide (1.5 g, 5.63 mmol) in dry methanol (100 mL) with acetic acid (9 mL) to adjust the pH to 3, was added sodium cyanoborohydride (1.8 g, 28.65 mmol). The reaction mixture was stirred for five hours at room temperature. A saturated sodium bicarbonate solution (100 mL) and water (100 mL) was added and the resulting mixture was stirred for one hour. The reaction mixture was extracted with ethyl acetate (2 x 200 mL); the pH (10-11) of the aqueous layer was adjusted to 8 by adding concentrated HCl (~15 mL) and a second extraction with ethyl acetate (2 x 200 mL) was performed. The organic extracts were combined, dried over $MgSO_4$, filtered and concentrated *in vacuo* to give a mixture of the product containing about 20%

of starting material. This solid was dissolved in dry methanol (100 mL) containing acetic acid (6 mL) and $NaBH_3CN$ (0.5 g) was added; the reaction mixture was stirred at room temperature for one day. After addition of a saturated bicarbonate solution (100 mL), the pH was adjusted to 5-6 by addition of sodium carbonate and the resulting solution was extracted with ethyl acetate (3 x 150 mL). The combined organic extracts were dried over $MgSO_4$, filtered and concentrated *in vacuo* to give the title compound as a white solid (1.32 g, 87% yield). $^1H$ NMR (300 MHz, DMSO-$d_6$); δ 1.31 (s, 9H); 2.36 (s, 3H); 3.82 (s, 2H); 4.75 (br s, 1H); 7.05 (m, 1H); 7.14-7.20 (m, 2H); 7.35 (d, 1H, J = 8 Hz); 8.21 (br s, 1H).

(*tert*-Butoxy)-N-{[7-chloro-4-oxo-2-(pyrrolidinylcarbonyl)(3-hydroquinolyl)]-N-[(2-methylthiophenyl)methyl]carbonylamino)carboxamide.

To a stirred solution of 7-chloro-4-oxo-2-(pyrrolidinylcarbonyl)hydroquinoline-3-carboxylic acid, Example 1, (1.55 g, 4.83 mmol) in dry THF (25 mL) under nitrogen was added CMC (3.08 g, 7.27 mmol). The reaction mixture was stirred for 10 minutes and then a solution of (*tert*-butoxy)-N-{[(2-methylthiophenyl)methyl]amino}carboxamide (1.30 g, 4.84 mmol) in dry THF (30 mL) was added. The reaction mixture was stirred at room temperature for 22 hours and filtered through diatomaceous earth. The filter cake was washed with THF several times and these washes were combined with the initial filtrate and concentrated in *vacuo* to give a yellow solid. This material was dissolved in DCM (200 mL) and washed with a saturated bicarbonate solution (4 x 100 mL). The organic material was dried on $MgSO_4$, filtered and concentrated *in vacuo* to give the title product as a yellow solid (2.35g, 85% yield). $^1H$ NMR (300 MHz, DMSO-$d_6$): δ 1.00-1.45 (m, 9H + 4H); 1.70-1.95 (m, 4H); 2.49(s, 3H); 3.20-3.50 (m, 2H + NH); 7.10-7.65 (m, 6H); 8.23 (d, 1H, J = 8 Hz).

7-Chloro-4,10-dihydroxy-2-[(2-methylthiophenyl)methyl]-2-hydropyridazino[4,5-*b*]quinoline-1-one.

To a stirred solution of (*tert*-butoxy)-N-{[7-chloro-4-oxo-2-(pyrrolidinylcarbonyl)(3-hydroquinolyl)]-N-[(2-methylthiophenyl)methyl]-carbonylamino)carboxamide (0.857 g, 1.5 mmol) in dry THF (60 mL) under nitrogen was added methanesulfonic acid (5.25 mL, 80.90 mmol). The reaction mixture was stirred at room temperature for 24 hours and ice water (250 mL) was added to generate a light yellow precipitate. The mixture was stirred for 2 hours and filtered to separate the solid which was washed with water and diethyl ether and then air dried. The solid was washed with methanol (4 x 40 mL) and diethyl ether and dried overnight at 45 *°C in vacuo* to give the title compound (0.352 g, 59% yield) as a light yellow solid, mp 290-292 °C. This powder contained -15% of the sulfoxide (ratio calculated by NMR). $^1H$ NMR (300 MHz, DMSO-$d_6$): δ 2.50-2.52 (s, 3H + DMSO); 2.80 (s, 3H from sulfoxide); 5.10 (s, 2H); 6.93 (d, 1H, *J* = 7.5 Hz); 7.11 (t, 1H, *J* = 7.5 Hz); 7.24-7.37 (m, 2H); 7.45 (d, 1H, *J*= 8 Hz); 8.05 (s, 1H); 8.15 (d, 1H, *J* = 8.5 Hz); 11.99 (s, 1H); 12.66 (s, 1H). Calc'd. for $C_{19}H_{14}ClN_3O_3S.0.35\ H_2O$: C, 56.53; H, 3.65; N, 10.35; Found: C, 56.53; H, 3.62; N, 9.92.

**Example 8:** 7-Chloro-2-(4-(2,6-dimethoxy)methylbenzoate)-4-hydroxy-1,2,5,10-tetrahydropyridazino[4,5-*b*]quinoline-1,10-dione. 4-Carbomethoxy-2,4-dimethoxybenzyl bromide:

To a solution of methyl 2,6-dihydroxy-4-methylbenzoate (1.98 g, 10.9 mmol) in DMF (15 mL) was added potassium carbonate (6.9 g, 50 mmol) and dimethyl sulfate (5.5 mL, 58 mmol). The mixture was vigorously stirred for 18 hr under nitrogen. The reaction mixture was quenched by addition of water (50 mL), extracted with diethyl ether (50 mL) and the organic phase washed three times with water (100 mL) and then with brine. The organic phase was dried over $MgSO_4$ and evaporated to yield 2.0 g of an off-white solid. Chromatography on silica gel with 25% ethyl acetate in DCM afforded 1.9 g of a white solid. The solid was recrystallized from refluxing hexanes (40 mL), and a white solid (1.05 g) was collected by vacuum filtration. A portion of this material (0.80 g, 3.8 mmol) was dissolved in carbon tetrachloride (20 mL), and *N*-bromosuccinimide (680 mg, 3.8 mmol) was added. The solution was photolyzed with a sunlamp for 30 min. and the solids were filtered off. The filtrate was diluted with ethyl acetate and washed with water. The organic layer was dried over $MgSO_4$ and evaporated. This gave a yellow oil (1.37 g, >100%). This was chromatographed on silica gel with 25% ethyl acetate in hexanes to afford the title compound as a slightly yellow oil. (400 mg, 36%). $^1H$ NMR (300 MHz, DMSO-$d_6$): δ 7.31 (s, 2H); 6.98 (s, 2H); 3.80 (s, 6H); 3.77 (s, 3H).

7-Chloro-2-(4-(2,6-dimethoxy)methylbenzoate)-4-hydroxy-1,2,5,10-tetrahydropyridazino[4,5-*b*]quinoline-1,10-dione

The title compound was prepared from 4-carbomethoxy-2,4-dimethoxybenzyl bromide by a procedure analogous to that described in Example 4. $^1H$ NMR (300 MHz, DMSO-$d_6$): δ 12.63 (s, 1H); 11.94 (s, 1H); 8.15 (d, *J*= 8.7 Hz, 1H); 8.03 (s, 1H); 7.43 (d, *J*= 7.5 Hz, 1H); 6.64 (s, 2H); 5.10 (s, 2H); 3.73 (s, 3H); 3.72 (s, 6H). Calc'd for $C_{22}H_{18}ClN_3O_7$•0.2 $H_2O$: C, 55.58; H, 3.90; N, 8.84; Found; C, 55.44, 55.78; H, 3.83, 3.84; N, 8.60, 8.63

**Example 9:** 7-Chloro-4-hydroxy-2-(4-*iso*-propoxycarbonylbenzyl)-1,2,5,10-tetrahydropyridazino[4,5-*b*]quinoline-1,10-dione.

To a solution of *iso*-propyl-4-formylbenzoate (1.40g, 7.28 mmol) in THF (30 mL) was added *tert*-butyl carbazate (960 mg, 7.28 mmol) and conc. HCl (3 drops). This solution was stirred for 24 h, and the THF was removed *in vacuo*. The solid was triturated with hexanes and the off-white solid was filtered to give methylethyl-4-(2-aza)-2-(*tert*-butoxy) carbamoylamino-vinylbenzoate (2.0 g, 6.53 mmol, 90%) which was used without further purification in the next step.

To a solution of methylethyl-4-(2-aza)-2-(*tert*-butoxy)carbamoylamino-vinylbenzoate (2.0 g, 6.53 mmol) in methanol (30 mL) was added acetic acid (4.5 mL) followed by sodium cyanoborohydride (2.1 g, 32 mmol). The mixture was heated to 65 °C for 2 h, and then cooled to room temperature. The mixture was diluted with water and the methanol was removed *in vacuo*. The mixture was extracted with ethyl acetate and the separated organic phase was washed with saturated aqueous $NaHCO_3$, $H_2O$, brine and then dried over $Na_2SO_4$. After filtration, the solvent was evaporated to afford methylethyl-4-({[(*tert*-butoxy)carbonylamino]amino}methyl)benzoate as a clear oil (2.1 g, 6.8 mmol, >100%). This material was used without further purification in the next step.

To a stirred slurry of 7-chloro-4-oxo-2-(pyrrolidinylcarbonyl)hydroquinoline-3-carboxylic acid, Example 1, (1.04 g, 3.25 mmol) in THF (20 mL) was added CMC (2.75 g, 6.50 mmol). To this canary yellow mixture was added a solution of methylethyl-4-({[(*tert*-butoxy)carbonylamino]amino}methyl)benzoate as a clear oil from the previous step (1.0 g, 3.25 mmol) and *N,N*-dimethyl-aminopyridine (59 mg, 487 μmol) in THF (15 mL). The mixture was refluxed for 3 h then cooled and filtered. The filtrate was concentrated and filtered through a small column of silica gel (ca. 7.5% MeOH/$CH_2Cl_2$) to afford N-[(*tert*-butoxy)carbonylamino][7-chloro-4-oxo-2-(pyrrolidinylcarbonyl)(3-hydroquinolyl)]-N-(4-carbo-iso-propoxybenzyl)carboxamide as a tan solid; this material was used in the next reaction without further purification.

To a stirred solution of the material from the previous step (3.25 mmol theor.) in THF (50 mL) was added methanesulfonic acid (6.0 mL, 92 mmol). This solution was stirred at rt. for 4 h, at which time the THF was evaporated and $H_2O$ was added to induce precipitation of the product. The material was filtered to yield a pale yellow solid. The solid was sonicated in 40 mL of a 20% MeOH/$Et_2O$ solution for 20 min, filtered and dried at 30 °C under 500 mTorr overnight to afford the title compound as a tan solid (700 mg, 1.59 mmol, 69% for two steps). $^1H$ NMR (300 MHz, DMSO-$d_6$) δ 12.70 (br s, 1H); 11.96 (s, 1H); 8.15 (d, J= 8.7 Hz, 1H); 8.03 (d, *J* = 1.8 Hz, 1H); 7.90 (d, *J* = 8.1 Hz, 2H); 7.46 (s, 1H); 7.42 (d, *J* = 8.4 Hz, 2H); 5.18 (s, 2H); 5.12 (p, *J* = 6.3 Hz, 1H); 1.30 (d, *J* = 6.0 Hz, 6H); Calc'd. for $C_{22}H_{18}O_5N_3Cl{\bullet}0.5H_2O$: C, 59.47; H, 4.20; N, 9.46. Found: C, 59.56, 59.15; H, 4.14, 4.13; N, 9.48, 9.42.

**Example 10:** 7-Chloro-4-hydroxy-2-(3-methoxycarbonylbenzyl)-1,2,5,10-tetrahydropyridazino[4,5-*b*]quinoline-1,10-dione.

The title compound was made from methyl 3-(bromomethyl)benzoate according to the procedure of Example 4. After air drying the title compound (0.327 g, 100%) was obtained as a white solid. $^1H$ NMR (300 MHz, DMSO-$d_6$) δ 12.62 (br s, 1H); 8.15 (d, *J* = 8.7 Hz, 1H); 8.03 (d, *J* = 1.8 Hz, 1H); 7.93 (s, 1H); 7.87 (d, *J* = 7.8 Hz, 1H); 7.62 (d, *J* = 7.8 Hz, 1H); 7.50 (t, *J* = 7.8 Hz, 1H); 7.44 (dd, *J* = 2.1, 8.7 Hz, 1H); 5.17 (s, 2H); 3.84 (s, 3H).

**Example 11:** 7-chloro-4-hydroxy-2-(4-dimethylaminocarboxamide)phenylmethyl-1,2,5,10-tetrahydropyridazino[4,5-*b*] quinoline-1,10-dione. (*tert*-Butoxy)-N-{[(4-nitrophenyl)methyl]amino}carboxamide.

To a solution of 4-nitrobenzylbromide (15 g, 69 mmol) in DMF (100 mL) was added *tert*-butylcarbazate (70 g, 529 mmol) and potassium carbonate (10 g, 75 mmol). The mixture was stirred and heated to 90 °C for 2 hours under nitrogen. At that time, the mixture was cooled, diluted with diethyl ether (400 mL) and washed with water (5 x 200 mL) and brine (100 mL). The organic phase was dried over $MgSO_4$, filtered and reduced by rotary evaporation to an oily brown solid. The residual DMF and *tert*-butylcarbazate were remove by kugelrohr distillation at 90 °C and 50 mTorr. The residual brown solid was purified by chromatography on silica gel, eluting with 10% ethyl acetate in DCM to give the title compound (15 g, 55 mmol, 79%) as a yellow solid.

(*tert*-Butoxy)-N-{[7-chloro-4-oxo-2-(pyrrolidinylcarbonyl)(3-hydroquinolyl)][(4-nitrophenyl)methyl]amino}carboxamide

A suspension of 7-chloro-4-oxo-2-(pyrrolidinylcarbonyl)hydroquinoline-3-carboxylic acid, Example 1, (12 g, 38 mmol) and CMC (32 g, 76 mmol) in THF (800 mL) was heated to reflux and stirred with an "over-head" stirrer under nitrogen. Five minutes after heating began, a solution of (*tert*-butoxy)-N-{[(4-nitrophenyl)methyl]amino}carboxamide (10 g, 38 mmol) and DMAP (0.5 g, 4 mmol) in THF (100 mL) was added. The mixture was refluxed for 4 hours and then allowed to cool. The solids were removed by vacuum filtration and discarded. The filtrate was reduced by rotary evaporation and the residue was purified by chromatography on silica gel eluting with a gradient of 3 to 7 % methanol in chloroform to give the title compound (14.5 g, 26 mmol, 68 %) as a yellow solid.

N- {[4-aminophenyl)methyl][7-chloro-4-oxo-2-(pyrrolinylcarbonyl)(3-hydroquinolyl)]amino}(*tert*-butoxy)carboxamide

(*tert*-Butoxy)-N-{[7-chloro-4-oxo-2-(pyrrolidinylcarbonyl)(3-hydroquinolyl)][(4-nitrophenyl)methyl]amino}carboxamide (6.9 g, 12 mmol) was dissolved in $CH_2Cl_2$ (1 L). 1,1'-Dioctadecyl-4,4'-bipyridinium dibromide (500 mg, 600 μmol) was added, followed by water (10 mL). This mixture was heated to 35 °C under nitrogen, at which time a solution of sodium dithionate (25.3 g, 145 mmol) and potassium carbonate (8.4 g, 60.5 mmol) in water (150 mL) was added. After 4 h, a second batch of sodium dithionate (25.4 g) and potassium carbonate (60.5 g) in water (150 mL) was added. The reaction was stirred an additional 18 h, then cooled to room temperature. The organic phase was separated, the aqueous was extracted with chloroform (100 mL), and the combined organic phases were washed with brine (50 mL), dried over anhydrous $MgSO_4$, and concentrated by rotary evaporation. This material was chromatographed on silica gel with 5% methanol in chloroform to afford the title compound (4.5 g, 8.33 mmol, 69%) as a yellow solid.

(Dimethylamino)-N-[4-({[*tert*-butoxy)carbonylamino][7-chloro-4-oxo-2-(pyrrolidinylcarbonyl)(3-hydroquinolyl)]amino} methyl)phenyl]carboxamide

N-{[4-aminophenyl)methyl][7-chloro-4-oxo-2-(pyrrolinylcarbonyl)(3-hydroquinolyl)]amino}(*tert*-butoxy)carboxamide (1.0 g, 1.9 mmol) was dissolved in $CH_2Cl_2$ (50 mL) and triethylamine (350 μL, 2.5 mmol) was added, followed by DMAP (10 mg, 100 μmol) and dimethylcarbamoyl chloride (200 μL, 2 mmol). The reaction was stirred for 50 h, and four 0.2 mL portions of additional dimethylcarbamoyl chloride were added during this time. The reaction mixture was washed with water and brine, dried over $MgSO_4$, and chromatographed on silica gel with 5% methanol in DCM to afford the title compound (690 mg, 1.13 mmol, 61%) as a yellow solid.

7-chloro-4-hydroxy-2-(4-dimethylaminocarboxamide)phenylmethyl-1,2,5,10-tetrahydropyridazino[4,5-*b*]quinoline-1,10-dione.

(Dimethylamino)-N-[4-({[*tert*-butoxy)carbonylamino][7-chloro-4-oxo-2-(pyrrolidinylcarbonyl)(3-hydroquinolyl)] amino}methyl)phenyl]carboxamide (690 mg, 1.13 mmol) was dissolved in a mixture of methanesulfonic acid (5 mL) in THF (25 mL). The reaction was allowed to stir for 16 hours. The volatiles were removed by rotary evaporation and the residue was precipitated by addition of water (150 mL). The solid was collected by vacuum filtration and washed with water (2 x 50 mL) then with diethyl ether (50 mL). The solid was suspended in 25% methanol in diethyl ether (200 mL) and sonicated for 15 minutes. The solid was collected by vacuum filtration and dried *in vacuo* to give the title compound (430 mg, 970 μmol, 86%) as a yellow solid. $^1H$ NMR (300 MHz, DMSO-$d_6$): δ 12.61 (s, 1H); 11.91 (s, 1H); 8.27 (s, 1H); 8.15 (d, *J*= 8.7 Hz, 1H); 8.02 (s, 1H); 7.44-7.37 (m, 3H); 7.17 (d, *J* = 8.4 Hz, 2H); 5.02 (s, 2H); 2.90 (s, 6H). Calc'd for $C_{21}H_{18}ClN_5O_4$•0.15 $H_2O$•0.15 $CH_3SO_3H$: C, 55.59; H, 4.17; N, 15.32; Found: C, 55.76; H, 4.15; N, 15.03.

**Example 12:** 7-Chloro-4-hydroxy-2-(4-(*tert*-butyl)aminocarboxamide)phenylmethyl-1,2,5,10-tetrahydropyridazino [4,5-*b*]quinoline-1,10-dione.

2: N-[(*tert*-Butoxy)carbonylamino]-N-[(3-{[(*tert*-butyl)amino]carbony]amino}-phenyl)methyl][7-chloro-4-oxo-2-(pyrrolidinylcarbonyl)(3-hydroquinolyl)]-carboxamide.

To a mixture of N-(N-[(3-aminophenyl)methyl][7-chloro-4-oxo-2-(pyrrolidinylcarbonyl)-(3-hydroquinolyl)]carbonylamino)(*tert*-butoxy)carboxamide (450 mg, 830 μmol) prepared by the method of Example 61 using 3-nitrobenzyl bromide in $CH_2Cl_2$ (10 mL) was added *tert*-butylisocyanate (120 μL, 1.1 mmol) and triethylamine (300 μL, 2.1 mmol). The mixture was stirred for 20 h, at which time additional *tert*-butylisocyanate (100 μL) was added. This solution was then stirred overnight and the reaction was diluted with ethyl acetate and washed with saturated aqueous $NH_4Cl$, water and brine and then dried over $Na_2SO_4$. This material was used in the following step without purification.

[(*tert*-Butyl)amino]-N-{3-[(7-chloro-4-hydroxy-1,10-dioxo(1,2,5,10-tetrahydropyridazino[4,5-*b*]quinolin-2-yl))methyl] phenyl)carboxamide.

To a mixture of N-[(*tert*-butoxy)carbonylamino]-N-[(3-{[(*tert*-butyl)amino]carbonylamino} phenyl)methyl] [7-chloro-4-oxo-2-(pyrrolidinylcarbonyl)(3-hydro-quinolyl)]-carboxamide (theoretically 830 μmol) in THF (10 mL) was added a room temperature solution of methanesulfonic acid (2.2 mL, 33.2 mmol) in THF (7 mL). The resultant yellow solution was stirred overnight, water was added and the solid collected by filtration. This solid was sonicated in 15 mL of a 10% methanol in diethyl ether solution and the remaining solid was collected and dried at 30 °C and 50 mTorr for 3 h. This afforded the title compound (230 mg, 490 μmol, 59% for two steps) as a tan solid. $^1H$ NMR (300 MHz, DMSO-$d_6$): δ 12.69 (br s, 1H); 11.94 (br s, 1H); 8.24 (s, 1H); 8.14 (d, *J*= 8.7 Hz, 1H); 8.03 (s, 1H); 7.44 (d, *J* = 8.4 Hz, 1H); 7.30 (d,

*J* = 8.1 Hz, 1H); 7.22 (s, 1H); 7.15 (dd, *J* = 7.8, 7.8 Hz, 1H); 6.81 (d, *J* = 7.5 Hz, 1H); 5.91 (s, 1H); 5.04 (s, 2H); 1.26 (s, 9H). MS (CI) *m/z* 468/470.

**Example 13:** 7-chloro-4-hydroxy-2-(4-pyrrolidinylcarboxamide)phenylmethyl-1,2,5,10-tetrahydropyridazino[4,5-*b*]quinoline-1,10-dione.

The title compound was prepared by the method described Example 11 from N-{[4-aminophenyl)methyl][7-chloro-4-oxo-2-(pyrrolinylcarbonyl)(3-hydroquinolyl)]amino}(*tert*-butoxy)carboxamide (Example 61) and 1-pyrrohdinecarbonyl chloride. $^1$H NMR (300 MHz, DMSO-$d_6$): δ 12.60 (s, 1H); 11.91 (s, 1H); 8.15 (d, *J* = 8.4 Hz, 1H); 8.09 (s, 1H); 8.02 (s, 1H), 7.44 (d, *J* = 8.4 Hz, 3H); 7.17 (d, *J* = 8.4 Hz, 2H); 5.02 (s, 2H); 3.45-3.60 (m, 4H); 1.70-1.90 (m, 4H). Calc'd for $C_{23}H_{20}ClN_5O_4$•1.3 $CH_3SO_3H$: C, 57.51; H, 5.00; N, 13.80. Found: C, 57.57; H, 5.18; N, 13.81.

**Example 14:** 7-chloro-4-hydroxy-2-(3-dimethylaminocarboxamide)phenylmethyl-1,2,5,10-tetrahydropyridazino[4,5-*b*]quinoline-1,10-dione.

The title compound was prepared by the method described Example 11 from N-{[3-aminophenyl)methyl][7-chloro-4-oxo-2-(pyrrolinylcarbonyl)(3-hydroquinolyl)]amino}(*tert*-butoxy)carboxamide (Example 62) and dimethylcarbamoyl chloride. $^1$H NMR (300 MHz, DMSO-$d_6$): δ 12.68 (s, 1H); 11.96 (s, 1H); 8.28 (s, 1H); 8.15 (d, *J* = 8.7 Hz, 1H); 8.02 (s, 1H); 7.30-7.50 (m, 3H); 7.17 (t, *J* = 7.8 Hz, 1H); 6.87 (d, *J* = 7.5Hz, 1H); 5.05 (s, 2H); 2.89 (s, 6H). Calc'd for $C_{21}H_{18}ClN_5O_4$•0.10 $H_2O$•0.35 $CH_3SO_3H$: C, 53.95; H, 4.16; N, 14.74. Found: C, 54.07; H, 4.13; N, 14.58.

**Example 15:** 7-Chloro-4-hydroxy-2-(3-pyrrolidinylcarboxamide)phenylmethyl-1,2,5,10-tetrahydropyridazino[4,5-*b*]quinoline-1,10-dione.

The title compound was prepared by the method described Example 11 from N-{[3-aminophenyl)methyl][7-chloro-4-oxo-2-(pyrrolinylcarbonyl)(3-hydroquinolyl)]amino}(*tert*-butoxy)carboxamide (Example 62) and 1-pyrrolidinecarbonyl chloride. $^1$H NMR (300 MHz, DMSO-$d_6$): δ 12.67 (s, 1H); 11.94 (s, 1H); 8.10-8.00 (m, 3H); 7.35-7.45 (m, 3H); 7.17 (t, *J*= 7.8 Hz, 1H); 6.86 (d, J = 7.5 Hz, 1H); 5.05 (s, 2H); 1.82 (s, 4H); signal at 3.33 is obscured by water. Calc'd for $C_{23}H_{20}ClN_5O_4$•0.15 $H_2O$•0.1 $CH_3SO_3H$: C, 58.02; H, 4.36; N, 14.65. Found: C, 58.19; H, 4.41; N, 14.40.

**Example 16:** 7-Chloro-4-hydroxy-2-(3-aminomethylphenyl)-1,2,5,10-tetrahydropyridazino[4,5-*b*]quinoline-1,10-dione.

The title compound was prepared from 3-nitrobenzylbromide by a procedure analogous to that described in Example 11. $^1$H NMR (300 MHz, DMSO-$d_6$): δ 11.93 (s,1H); 8.15 (d, *J* = 8.7 Hz, 1H); 8.02 (s, 1H); 7.44 (d, *J* = 8.4 Hz, 1H); 6.44-6.42 (m, 3H); 4.96 (s, 2H).

**Example 17:** 7-chloro-4-hydroxy-2-(4-phenylaminocarboxamide)phenylmethyl-1,2,5,10-tetrahydropyridazino[4,5-*b*]quinoline-1,10-dione.

The title compound was prepared by the method described in Example 12 from N-{[4-aminophenyl)methyl][7-chloro-4-oxo-2-(pyrolinylcarbonyl)(3-hydroquinolyl)]amino}(*tert*-butoxy)carboxamide (Example 61) and phenyl isocyanate. $^1$H NMR (300 MHz, DMSO-$d_6$): 12.63 (s, 1H); 11.92 (s,1H); 8.65 (d, *J* = 12.3 Hz, 2H); 8.15 (d, *J* = 8.7 Hz, 1H); 8.02 (s, 1H); 7.30-7.50 (m, 5H); 7.20-7.30 (m, 4H); 6.95 (t, *J*= 7.2 Hz, 1H); 5.04 (s. 2H). Calc'd for $C_{25}H_{18}ClN_5O_4$: C, 61.54; H, 3.72; N, 14.35. Found: C, 61.50; H, 3.79; N, 13.99.

**Example 18:** 7-chloro-4-hydroxy-2-(3-phenylaminocarboxamide)phenylmethyl-1,2,5,10-tetrahydropyridazino[4,5-*b*]quinoline-1,10-dione.

The title compound was prepared by the method described in Example 12 from N-{[3-aminophenyl)methyl][7-chloro-4-oxo-2-(pyrrolinylcarbonyl)(3-hydroquinolyl)]amino}(*tert*-butoxy)carboxamide (Example 62) and phenyl isocyanate. $^1$H NMR (300 MHz, DMSO-$d_6$): δ 12.69 (s, 1H); 11.96 (s, 1H); 8.69 (s, 1H); 8.59 (s, 1H); 8.15 (d, *J* = 8.4Hz, 1H); 8.03 (s, 1H); 7.35-7.45 (m, 4H); 7.20-7.30 (m, 4H); 6.9-7.0 (m, 2H); 5.08 (s, 2H). Calc'd for $C_{25}H_{18}ClN_5O_4$•0.15 $H_2O$•0.05 $CH_3SO_3H$: C, 60.73; H, 3.76; N, 14.14. Found: C, 60.77; H, 3.82; N, 13.96.

**Example 19:** 7-chloro-4-hydroxy-2-(3-cyclohexylaminocarboxamide)phenylmethyl-1,2,5,10-tetrahydropyridazino[4,5-*b*]quinoline-1,10-dione.

The title compound was prepared by the method described in Example 12 from N-{[3-aminophenyl)methyl]

[7-chloro-4-oxo-2-(pyrrolinylcarbonyl)(3-hydroquinolyl)]amino}(*tert*-butoxy)carboxamide (Example 62) and cyclohexyl isocyanate. $^1$H NMR (300 MHz, DMSO-$d_6$): δ 8.18 (d, *J* = 8.7 Hz, 1H); 8.06 (s, 1H); 7.44 (d, *J* = 8.4 Hz, 1H); 7.33 (d, *J* = 8.12 Hz, 1H): 7.27 (s, 1H); 7.17 (dd, *J* = 7.8, 7.8 Hz, 1H); 6.85 (d, *J* = 7.5 Hz, 1H); 5.07 (s, 2H); 3.41 (m, 1H); 1.79-1.51 (m, 5H); 1.32-1.08 (m, 5H).

**Example 20:** 7-Chloro-4-hydroxy-2-(4-iodophenylmethyl)-1,2,5,10-tetrahydropyridazino[4,5-*b*]quinoline-1,10-dione.

The title compound was synthesized by the method of Example 12 using 4-iodobenzyl bromide as the starting material. $^1$H NMR (300 MHz, DMSO-$d_6$) δ 12.67 (br s, 1 H); 11.95 (br s, 1 H); 8.13 (br d, *J* = 6.6 Hz, 1 H); 8.04 (s, 1 H); 7.69 (d, *J* = 7.5 Hz, 2 H); 7.44 (d, *J* = 6.6 Hz, 1 H); 7.11 (d, *J* = 7.5 Hz, 2 H); 5.05 (s, 2 H).

**Example 21:** 7-Chloro-4-hydroxy-2-(4-(3-proparginol)phenyl)-1,2,5,10-tetrahydropyridazino[4,5-*b*]quinoline-1,10-dione.

N[(*tert*-Butoxy) carbonylamino] [7-chloro- 4-oxo- 2-(pyrrolidinylcarbonyl) (3-hydroquinolyl)] -N-[(4-iodophenyl) methyl]carboxamide, Example 20, (200 mg, 0.3 mmol), propargyl alcohol (0.026 mL, 0.43 mmol), and BHT (1 mg, 0.0045 mmol) in $CHCl_3$ (4 mL) were treated sequentially with dichlorobis(triphenylphosphine)palladium(II) (8 mg, 0.012 mmol), copper(I) iodide (5.2 mg, 0.028 mmol), and triethylamine (0.12 mL, 0.92 mmol). After stirring for 20 hours, the brown solution was cooled to room temperature, diluted with water, extracted with $CH_2Cl_2$, dried ($Na_2SO_4$) and concentrated to a yellow solid. Flash chromatography ($SiO_2$, 96:4 MeOH-$CH_2Cl_2$) gave a white solid (125 mg, 72%). This material was taken up in THF (2.5 mL), and treated dropwise with methanesulfonic acid (0.5 mL, 7.8 mmol). After stirring for 20 hours, the yellow solution was concentrated then treated with ice water. The resulting precipitate was collected by suction filtration, suspended in methanol, sonicated for 1 hour, and dried *in vacuo* to yield the title compound as a yellow solid, 40 mg (49%). $^1$H NMR (300 MHz, DMSO-$d_6$) δ 12.64 (br s, 1 H); 11.92 (br s, 1 H); 8.15 (d, *J* = 8.4 Hz, 1 H); 8.03 (s, 1 H); 7.45-7.42 (m, 2 H); 7.39 (d, *J* = 8.1 Hz, 1 H); 7.29 (d, *J* = 8.1 Hz, 1 H); 5.11 (s, 2 H); 4.28 (s, 2H).

**Example 22:** 7-Chloro-4-hydroxy-2-{[3-(piperazinylcarbonyl)phenyl]methyl}-1,2,5,10-tetrahydropyridazino[4,5-*b*]quinoline-1,10-dione methanesulfonate. 3-{2-Aza-2-[(*tert*-butoxy)carbonylamino]vinyl}benzoic acid.

To a stirred solution of 3-formylbenzoic acid (3.0 g, 19.9 mmol) in THF (100 mL) was added *tert*-butyl carbazate (2.64 g, 20.8 mmol) and the mixture was stirred 48 hours. The THF was removed *in vacuo* and the residual solid triturated with hexanes to give the title compound as a white solid (5.0 g, 95%). $^1$H NMR (300 MHz, DMSO-$d_6$): δ 1.49 (s, 9H); 7.54 (t, 1H, J = 7.8 Hz); 7.80 (d, 1H, J = 7.8 Hz); 7.93 (d, 1H, J = 7.8 Hz); 8.07 (s, 1H); 8.20 (s, 1H); 11.03 (br s, 1H).

N-{1-Aza-2-[3-(piperazinylcarbonyl)phenyl]vinyl}-(*tert*-butoxy)carboxamide.

A stirred suspension of 3-{2-aza-2-[(*tert*-butoxy)carbonylamino]vinyl}benzoic_acid (0.80 g, 3 mmol) in DCM (15 mL) was cooled in an ice-bath and to this was added 1-(3-dimethylaminopropyl)-3-ethylcarbodiimide hydrochloride (0.65 g, 3.45 mmol). The mixture was stirred for five minutes and to this was added *tert*-butyl 1-piperazinecarboxylate (0.56 g, 3 mmol) and DMAP (0.02 g, 0.1 mmol) as a solution in DCM (3 mL). The reaction was stirred for 24 hours, and then poured into a separatory funnel along with an additional portion of DCM (50 mL) and water (20 mL). The organic layer was washed with water, sodium bicarbonate (sat. aqueous, 20 mL) and sodium chloride (sat. aqueous, 20 mL). The organic layer was collected, dried over $MgSO_4$ and removed *in vacuo* to give the title compound as an off-white solid (1.2 g, 93%). $^1$H NMR (300 MHz, DMSO-$d_6$): δ 1.40 (s, 9H); 1.46 (s, 9H); 3.33-3.58 (m, 8H); 7.35 (d, 1H, J = 7.5 Hz); 7.48 (t, 1H, J = 7.5 Hz); 7.62 (s, 1H); 7.67 (d, 1H, J = 7.5 Hz); 8.01 (s, 1H); 11.01 (br s 1H).

(*tert*-Butoxy)-N-[3-(1-piperazinylcarbonyl)phenyl]methyl)amino]carboxamide.

A mixture of 10 % palladium on carbon (0.200 g) and N-{1-aza-2-[3-(piperazinylcarbonyl)phenyl]vinyl}-(*tert*-butoxy)carboxamide (1.2 g, 2.77 mmol) in methanol (40 mL) was hydrogenated (40 psi) at room temperature for 2 hours. The reaction was filtered through diatomaceous earth and the filtrate evaporated under reduced pressure to give the title compound as an oil (0.93 g, 77%). Residual methyl alcohol could be removed by adding DCM (20 mL) and subsequently removing *in vacuo*. $^1$H NMR (300 MHz, DMSO-$d_6$): δ 1.36 (s, 9H); 1.40 (d, 9H); 3.33-3.58 (m, 8H); 3.87 (br s, 2H); 4.88 (br s, 1H); 7.27 (m, 1H); 7.33 (s, 1H); 7.37 (m, 2H); 8.24 (br s, 1H).

N-[(*tert*-Butoxy)carbonylamino][7-chloro-4-oxo-2-(pyrrolidinylcarbonyl)(3-hydroquinolyl]-N-{[3-(1-piperazinylcarbonyl)phenyl]methylcarboxamide.

To a stirred slurry of 7-chloro-4-oxo-2-(pyrrolidinylcarbonyl)hydroquinoline-3-carboxylic acid, Example 1, (0.65 g, 2.04 mmol) in DCM (40 mL) was added 1-(3-dimethylaminopropyl)-3-ethylcarbodiimide hydrochloride (0.44 g, 2.34 mmol) and the reaction was stirred for five minutes. To this mixture was rapidly added a solution of (*tert*-butoxy)-N-[3-(1-piperazinylcarbonyl)phenyl]methyl) amino]carboxamide (0.93 g, 2.14 mmol) and DMAP (0.02 g, 0.1 mmol) in DCM (10 mL), and the mixture was refluxed for four hours. The reaction was cooled, diluted with DCM (50 mL). The DCM was extracted with water (1 x 20 mL), sodium bicarbonate (sat. aqueous, lx 20 mL) and sodium chloride (sat. aqueous, 1 x 20 mL). The organic layer was dried over $MgSO_4$ and the solvent removed *in vacuo* to give the title compound as a yellow foam (1.3 g, 86%) which was used in the next reaction without further purification.

7-Chloro-4-hydroxy-2-{[3-(piperazinylcarbonyl)phenyl]methyl}-1,2,5,10-tetrahydropyridazino[4,5-*b*]quinoline-1,10-dione methanesulfonate.

To a stirred solution of N-[(*tert*-butoxy)carbonylamino][7-chloro-4-oxo-2-(pyrrolidinylcarbonyl)(3-hydroquinolyl]-N-{[3-(1-piperazinylcarbonyl)phenyl]methylcarboxamide (1.3 g, 1.62 mmol) in THF (30 mL) was added methanesulfonic acid (4 mL) and the reaction was stirred overnight. The volatiles were removed *in vacuo* and to the residual oil was added diethyl ether (200 mL). The mixture was stirred for 10 minutes and then allowed to settle into two layers, an etheral layer and layer of brown oil. The ether was decanted away and to the brown oil was added water (5 mL), followed by sodium chloride (sat. aqueous, 3 mL). After a short time, a precipitate formed and was collected by vacuum filtration. The precipitate was washed with a mixture of diethyl ether/methyl alcohol (5/1, 3 x 20 mL) and sonicated in 20 mL of 5/1 diethyl ether/methyl alcohol for fifteen minutes. The material was filtered, washed with diethyl ether and dried in *vacuo* to give the title compound (0.33 g, 44%) as an off-white powder. $^1H$ NMR (300 MHz, DMSO-$d_6$): δ 2.30 (s, 3H, C<u>H</u>$_3SO_3H$); 3.13 (m, 4H); 3.69 (m, 4H); 5.15 (s, 2H); 7.42 (m, 5H); 8.04 (s, 1H); 8.15 (d, 1H, J = 8.7 Hz); 9.16 (br s); 11.96 (br s); 12.70 (br s, 1H). MS (+CI) *m*/*z* 466/468.

**Examples 23 - 28:**

For Examples 23-28 below, intermediates of the hydrazine amides were prepared in a manner analogous to the preparation of 7-chloro-4-hydroxy-2-{[3-(piperazinylcarbonyl)phenyl]methyl}-1,2,5,10-tetrahydropyridazino[4,5-*b*]quinoline-1,10-dione methanesulfonate, Example 22. Cyclization of the hydrazine amides to give the title compounds was achieved by the following procedure:

To a stirred solution of hydrazine amide (1.62 mmol) in THF (30 mL) was added methanesulfonic acid (4 mL) and the reaction was stirred overnight. Volatiles were removed *in vacuo* and to the residual oil was added diethyl ether (200 mL). The mixture was stirred for 10 minutes and then allowed to settle into two layers, an etheral layer and layer of brown oil. The ether was decanted away and to the brown oil was added water (5 mL). After a short time, a precipitate formed and was collected by vacuum filtration. The precipitate was washed with a diethyl ether (3 x 20 mL) and then sonicated in 20 mL of 5/1 diethyl ether/methyl alcohol for fifteen minutes. The material was filtered, washed with diethyl ether and dried *in vacuo* to give the title compound.

**Example 23:** 7-Chloro-4-hydroxy-2-[4-(2-methoxy)ethylaminocarbonyl)-benzyl]-1,2,5,10-tetrahydropyridazino[4,5-*b*]quinoline-1,10-dione.

Yield of cyclization : 60%, off-white solid. $^1H$ NMR (300 MHz, DMSO-$d_6$): δ 3.25 (s, 3H); 3.39 (m, 2H); 3.67 (m, 2H); 5.15 (s, 2H); 7.35 (d, 2H, J = 8.1 Hz); 7.42 (d, 1H, J = 8.7 Hz); 7.80 (d, 2H, J = 8.1 Hz); 8.03 (s, 1H); 8.15 (d, 1H, J = 8.7 Hz); 8.50 (m, 1H); 11.94 (br s); 12.67 (br s, 1H). MS (+CI) *m*/*z* 455/457.

**Example 24:** 7-Chloro-4-hydroxy-2-[3-(methoxymethylaminocarbonyl)-benzyl]-1,2,5,10-tetrahydropyridazino[4,5-*b*]quinoline-1,10-dione.

Yield of cyclization : 50%, off-white solid. $^1H$ NMR (300 MHz, DMSO-$d_6$): δ 3.22 (s, 3H); 3.52 (s, 3H); 3.67 (m, 2H); 5.15 (s, 2H); 7.42 (m, 3H); 7.50 (m, 1H); 8.02 (s, 1H); 8.15 (d, 1H, J = 8.7 Hz); 11.94 (br s); 12.67 (br s, 1H). MS (+CI) *m*/*z* 441/443.

**Example 25:** 7-Chloro-4-hydroxy-2-[3-(methylaminocarbonyl)-benzyl]-1,2,5,10-tetrahydropyridazino[4,5-*b*]quinoline-1,10-dione.

Yield of cyclization : 50%, off-white solid. $^1H$ NMR (300 MHz, DMSO-$d_6$): δ 2.75 (d, 3H, J = 4.2 Hz); 5.15 (s, 2H); 7.42 (m, 2H); 7.71 (d, 1H, J = 6.9 Hz); 7.74 (s, 1H); 8.02 (s, 1H); 8.15 (d, 1H, J = 8.7 Hz); 8.43 (d, 1H, J = 4.2 Hz); 11.94 (br s); 12.66 (br s, 1H). MS (+CI) *m*/*z* 411/413.

**Example 26:** 7-Chloro-4-hydroxy-2-[3-(2-methoxy)ethylaminocarbonylbenzyl]-1,2,5,10-tetrahydropyridazino[4,5-*b*]quinoline-1,10-dione.

Yield of cyclization : 21%, off-white solid. $^1H$ NMR (300 MHz, DMSO-$d_6$): δ 3.24 (s, 3H); 3.40 (m, 4H); 5.15 (s, 2H); 7.42 (m, 3H); 7.71 (d, 1H, J = 6.9 Hz); 7.78 (s, 1H); 8.02 (s, 1H); 8.15 (d, 1H, J = 8.7 Hz); 8.53 (m, 1H, J = 4.2 Hz); 11.94 (br s); 12.69 (br s, 1H). MS (+CI) *m*/*z* 455/457.

**Example 27:** 7-Chloro-4-hydroxy-2-[3-(di-(2-methoxy)ethyl)aminocarbonylbenzyl]-1,2,5,10-tetrahydropyridazino[4,5-*b*]quinoline-1,10-dione.

Yield of cyclization : 42%, off-white solid. $^1H$ NMR (300 MHz, DMSO-$d_6$): δ 0.97 (m, 3H); 1.08 (m, 3H); 3.22-3.55 (br m 12H); 5.15 (s, 2H); 7.25 (m, 2H); 7.34 (m, 2H); 7.42 (d, 1H, J = 8.7 Hz); 8.03 (s, 1H); 8.15 (d, 1H, J = 8.7 Hz); 11.98 (br s); 12.70 (br s, 1H). MS (+CI) *m*/*z* 542/544.

**Example 28:** 7-Chloro-4-hydroxy-2-{[3-(piperazinylcarbonyl)phenyl]methyl}-1,2,5,10-tetrahydropyridazino[4,5-*b*]quinoline-1,10-dione.

Yield of cyclization : 40%, off-white solid. $^1H$ NMR (300 MHz, DMSO-$d_6$): δ 2.34 (s, 3H, $C\underline{H}_3SO_3H$); 3.16 (m, 4H); 3.69 (m, 4H); 5.15 (s, 2H); 7.42 (m, 5H); 8.03 (s, 1H); 8.15 (d, 1H, J = 8.7 Hz); 8.82 (br s); 11.97 (br s); 12.61 (br s, 1H). MS (+CI) *m*/*z* 466/468.

**Example 29:** 7-Chloro-4-hydroxy-2-[4-(morpholine-4-carbonyl)-benzyl]-1,2,5,10-tetrahydropyridazino[4,5-*b*]quinoline-1,10-dione. 4-{2-Aza-2-[(*tert*-butoxy)carbonylamino]vinyl}benzoic acid.

4-Carboxybenzaldehyde (2.0 g, 13.3 mmol) was added to THF (60 mL) and to this was added *tert*-butylcarbazate (1.76 g, 13.3 mmol), followed by aqueous hydrochloric acid (2 N, 2 drops from a Pasteur pipette). The reaction mixture was stirred for 16 hours and then the solvent was removed under reduced pressure. The residual solid was triturated with hexanes and collected to give a white solid (3.1 g, 89%). $^1H$ NMR (300 MHz, DMSO-$d_6$): δ 1.47 (s, 9H); 7.70 (d, 2H, J = 8.4 Hz); 7.94 (d, 2H, J = 8.4 Hz); 8.05 (s, 1H); 11.03 (br s, 1H); 12.98 (br s, 1H).

N-{1-Aza-2-[4-(morpholin-4-ylcarbonyl)phenyl]vinyl}-(*tert*-butoxy)carboxamide.

4-{2-Aza-2-[(*tert*-butoxy)carbonylamino]vinyl}benzoic acid (1.0 g, 3.78 mmol) was slurried in THF (10 mL) and to this was added CMC (1.67 g, 3.96 mmol), followed by morpholine (0.32 g, 3.78 mmol). The mixture was stirred overnight and a precipitate formed. The precipitate was collected and washed with THF (20 mL) to give the title compound as a white solid (0.80 g, 64%). This material was used in the following reaction without further purification. MS (+CI) *m*/*z* 334.

(*tert*-Butoxy)-N-({[4-(morpholin-4-ylcarbonyl)phenyl]methyl}amino) carboxamide.

N-{1-Aza-2-[4-(morpholin-4-ylcarbonyl)phenyl]vinyl}-(*tert*-butoxy)carboxamide (0.80 g, 2.4 mmol) was added to a Parr shaker bottle and slurried in methyl alcohol (30 mL). To this was added 10% palladium-on-carbon (~100 mg) and the reaction was hydrogenated at 40 psi for 4 hours. The catalyst was filtered on diatomaceous earth, washed with methyl alcohol (2 x 100 mL), and the solvents were removed *in vacuo* to give a oil. The oil was subjected to flash chromatography ($SiO_2$, ethyl acetate/hexanes as eluant) to give the title compound as an oil (0.46 g, 58%). $^1H$ NMR (300 MHz, DMSO-$d_6$): δ 1.37 (s, 9H); 3.22 (t, 2H, J = 5.1 Hz); 3.50 (t, 2H, J = 5.1 Hz); 3.52 (m, 4H); 3.88 (d, 2H, J = 4.5 Hz); 4.82 (m, 1H); 7.33 (d, 2H, J = 8.4 Hz); 7.38 (d, 2H; J = 8.4 Hz); 8.21 (br s, 1H).

N-[(*tert*-Butoxy)carbonylamino][7-chloro-4-oxo-2-(pyrrolidinylcarbonyl)(3-hydroquinolyl]-N-{[4-(morpholin-4-ylcarbonyl)phenyl]methyl}carboxamide.

To a stirred slurry of 7-chloro-4-oxo-2-(pyrrolidinylcarbonyl)hydroquinoline-3-carboxylic acid, Example 1, (0.47 g, 1.47 mmol) in THF (10 mL) was added CMC (0.71 g, 1.68 mmol) and the reaction was stirred for five minutes. To this mixture was added, *via* dropwise addition, a solution of (*tert*-butoxy)-N-({[4-(morpholin-4-ylcarbonyl)phenyl]methyl}amino) carboxamide (0.46 g, 1.40 mmol) and DMAP (0.005 g, 0.04 mmol) in THF (5 mL), and the mixture was then refluxed for 16 hours. The solution was filtered and the insolubles washed with THF (2 x 20 mL), water (2 x 20 mL) and diethyl ether (2 x 20 mL). The solids were collected to give the title compound as a white solid (0.42 g, 47%). This material was used in the following reaction without further purification. MS (-CI) *m*/*z* 637/638.

7-Chloro-4-hydroxy-2-{[4-(morpholin-4-ylcarbonyl)phenyl]methyl}-1,2,5,10-tetrahydropyridazino[4,5-*b*]quinoline-1,10-dione.

To a stirred solution of N-[(*tert*-butoxy)carbonylamino][7-chloro-4-oxo-2-(pyrrolidinylcarbonyl)(3-hydroquinolyl] -N-{[4-(morpholin-4-ylcarbonyl)phenyl]methyl}carboxamide (0.42 g, 0.65 mmol) in THF (20 mL) was added methanesulfonic acid (1.2 mL) and the reaction was heated to 70 °C for 1 hour. The reaction was then cooled to room temperature and stirred for 3 hours. The THF was removed under reduced pressure to yield an orange oil. To this was added diethyl ether (50 mL) and the reaction stirred for 10 minutes. The ether was carefully decanted to leave a thick orange oil. To this was added water (5 mL) and a fluffy yellow precipitate formed. The mixture was stirred for 10 minutes and then filtered. The solids were washed with water (10 mL) followed by diethyl ether (20 mL). The solids were collected and sonicated in methyl alcohol/diethyl ether (1:1, 5 mL) for 10 minutes. The solids were collected and dried under reduced pressure to give the title compound as a cream-colored solid (0.147 g, 49%). $^1$H NMR (300 MHz, DMSO-$d_\delta$): δ 3.33 (m, 4H); 3.51 (m, 4H); 5.14 (s, 2H); 7.36 (m, 4H); 7.43 (d, 1H, J = 8.7 Hz); 8.02 (s, 1H); 8.14 (d, 1H, J = 8.7 Hz); 11.94 (br s, 1H); 12. 67 (br s, 1H). MS (+CI) *m*/*z* 467/469.

**Example 30:** 7-Chloro-4-hydroxy-2-[4-(methylcarbonyl-amino)-benzyl]-1,2,5,10-tetrahydropyridazino[4,5-*b*]quinoline-1,10-dione.

The title compound was prepared by the method of Example 11, utilizing N-{[4-aminophenyl)methyl][7-chloro-4-oxo-2-(pyrrolinylcarbonyl)(3-hydroquinolyl)]amino}(*tert*-butoxy)carboxamide, Example 11, and acetyl chloride as the starting materials. $^1$H NMR (300 MHz, DMSO-$d_6$): d 12.55 (br s, 1H); 11.88 (br s, 1H); 9.89 (s, 1H); 8.15 (d, *J* = 8.7 Hz, 1H); 8.02 (s, 1H); 7.51 (d, *J* = 8.1 Hz, 2 H); 7.42 (d, *J* = 9.3 Hz, 1H); 7.22 (d, *J* = 8.4 Hz, 2H); 5.04 (s, 2H); 2.01 (s, 3H).

**Example 31:** 7-chloro-4-hydroxy-2-(4-isopropylcarboxamide)phenylmethyl-1,2,5,10-tetrahydropyridazino[4,5-*b*]quinoline-1,10-dione.

The title compound was prepared by the method of Example 11, utilizing N-{[4-aminophenyl)methyl][7-chloro-4-oxo-2-(pyrrolinylcarbonyl)(3-hydroquinolyl)]amino}(*tert*-butoxy)carboxamide, Example 11, and isobutyryl chloride as the starting materials. $^1$H NMR (300 MHz, DMSO-$d_6$): d 8.26 (s, 1H); 8.15 (d, *J*= 9.1 Hz, 1H); 8.02 (s, 1H); 7.43 (d, *J* = 9.7 Hz, 1H); 7.31 (d, *J* =8.1 Hz, 2H); 7.16 (d, *J* =8.0 Hz, 2H); 5.93 (m, 1H); 5.0 (s, 2H); 3.72 (m, 1H); 1.08 (s, 3H); 1.06 (s, 3H).

**Example 32:** 7-Chloro-4-hydroxy-2-[4-(methoxymethylcarbonyl-amino)-benzyl]-1,2,5,10-tetrahydropyridazino[4,5-*b*] quinoline-1,10-dione.

The title compound was prepared by the method of Example 11, utilizing N-{[4-aminophenyl)methyl][7-chloro-4-oxo-2-(pyrrolinylcarbonyl)(3-hydroquinolyl)]amino}(*tert*-butoxy)carboxamide, Example 11, and methoxyacetyl chloride as the starting materials. $^1$H NMR (300 MHz, DMSO-$d_6$): d 12.60 (br s, 1H); 11.95 (br s, 1H); 9.73 (s, 1H); 8.15 (d, J = 8.7 Hz, 1 H); 8.02 (d, J = 1.8 Hz); 7.60 (d, J = 8.7 Hz, 2H); 7.43 (dd, J = 1.8, 8.7 Hz, 1H); 7.24 (d, J = 8.4 Hz, 2H); 5.05 (s, 2H); 3.97 (s, 2H); 3.36 (s, 3H).

**Example 33:** 7-Chloro-4-hydroxy-2-[4-(*iso*-propylcarbonyl-amino)-benzyl]-1,2,5,10-tetrahydropyridazino[4,5-*b*]quinoline-1,10-dione.

The title compound was prepared by the method of Example 11, utilizing N-{[4-aminophenyl)methyl][7-chloro-4-oxo-2-(pyrrolinylcarbonyl)(3-hydroquinolyl)]amino}(*tert*-butoxy)carboxamide, Example 11, and isovaleryl chloride as the starting materials. $^1$H NMR (300 MHz, DMSO-$d_6$): d 12.61 (br s, 1H); 11.90 (br s, 1H); 8.14 (d, J = 8.7 Hz, 1H); 8.03 (s, 1H); 7.53 (d, *J* = 8.7 Hz, 2H); 7.43 (d, *J* = 9.1 Hz, 1H); 7.23 (d, *J* = 8.4 Hz, 2H); 5.04 (s, 2H); 2.15 (d, *J* = 6.9 Hz, 2H);

2.06 (m, 1H); 0.92 (s, 3H); 0.90 (s, 3H).

**Example 34:** 7-chloro-4-hydroxy-2-(4-(2-*iso*-propylphenyl)-carboxamide)phenylmethyl-1,2,5,10-tetrahydropyridazino [4,5-*b*]quinoline-1,10-dione.

The title compound was prepared by the method of Example 11, utilizing N-{[4-aminophenyl)methyl][7-chloro-4-oxo-2-(pyrrolinylcarbonyl)(3-hydroquinolyl)]amino}(*tert*-butoxy)carboxamide, Example 11, and 2-*iso*-propylphenyl isocyanate as the starting materials. $^1H$ NMR (300 MHz, DMSO-$d_6$): d 12.59 (br s, 1H); 11.91 (br s, 1H); 8.95 (s, 1H); 8.15 (d, *J* = 8.7 Hz, 1H); 8.02 (d, *J* = 1.5 Hz, 1H); 7.91 (s, 1H); 7.63 (m, 1H); 7.42 (m, 2H); 7.25 (m, 3H); 7.21 - 7.05 (m, 3H); 5.04 (s, 2H); 3.16 (m, 1H); 1.19 (s, 3H); 1.17 (s, 3H).

**Example 35:** 7-Chloro-4-hydroxy-2-(4-(2-methoxyethyl)aminocarboxamide)phenylmethyl-1,2,5,10-tetrahydropyridazino[4,5-*b*]quinoline-1,10-dione.

To a solution of 4-carboxybenzaldehyde (7.51 g, 50 mmol) in 50 mL warm THF was added *tert*-butylcarbazate (6.61 g, 50 mmol). This dissolved and a product began to precipitate; three drops of conc. HCl was added, and the reaction was stirred for 1.5 h. The reaction was diluted with ether (500 mL). The solid was collected by vacuum filtration, and washed with ether (100 mL). The hydrazone was obtained as a white solid (11.9 g, 90 % yield), and used without further purification.

To the hydrazone (1.57 g, 5.95 mmol) as a suspension in toluene (40 mL) was added diphenylphosphoryl azide (1.5 mL, 7 mmol) and triethylamine (0.83 mL, 6 mmol). The reaction was refluxed under $N_2$ for 2 h, then allowed to cool. 2-Methoxyethylamine (2.2 mL, 25 mmol) was added and allowed to react for 2 h, at which time the reaction was diluted with THF and washed with water and brine. The organic phase was dried over $MgSO_4$ and concentrated by rotary evaporation. The residue was dissolved in $CH_2Cl_2$ and chromatographed on silica gel eluting with ethyl acetate. The appropriate fractions were combined and concentrated by rotary evaporation. N-[1-aza-2-(4-{[(2-methoxyethyl)amino] carbonylamino}phenyl)vinyl][tert-butoxy)carboxamide was obtained as a white solid (1.08 g. 54 % yield). $^1$HNMR (DMSO-$d_6$) d 10.70 (s, 1H), 8.71 (s, 1H), 7.89 (s, 1H), 7.44-7.40 (m, 4H), 6.26 (t, *J* = 5.7 Hz, 1H), 3.40-3.36 (m, 2H), 3.28 (s, 3H), 3.28-3.22 (m, 2H),1.46 (s, 9H).

To a solution of N-[1-aza-2-(4-{[(2-methoxyethyl)amino]carbonylamino}-phenyl)vinyl][tert-butoxy)carboxamide (1.04 g, 3.1 mmol) in methanol (50 mL) was added ammonium formate (5.0 g, 79 mmol), followed by a slurry of 10 % Pd/C (300 mg, 0.3 mmol) in ethanol (5 mL). After 1.5 h the catalyst was removed by filtration through a pad of diatomaceous earth and filtrate was evaporated to dryness by rotary evaporation. The residue was partitioned between ethyl acetate and water, and the organic phase was washed with water and brine then dried over $MgSO_4$. Rotary evaporation gave the product hydrazine as a white solid (970 mg, 93 % yield). This material was used without further purification. $^1$HNMR (DMSO-$d_6$) d 8.46 (s, 1H), 8.18 (s, 1H), 7.30 (d, *J =* 8.4 Hz, 2H), 7.14 (d, *J =* 8.4 Hz, 2H), 6.15 (t, *J =* 5.7 Hz, 1H), 4.55 (s, 1H), 3.75 (s, 2H), 3.39-3.35 (m, 2H), 3.27 (s, 3H), 3.27-3.22 (m, 2H), 1.38 (s, 9H).

To a suspension of 7-chloro-4-oxo-2-(pyrrolidinylcarbonyl)hydroquinoline-3-carboxylic acid (Example 1) (1.06 g, 3.3 mmol) in $CH_2Cl_2$ (25 mL) was added 1-ethyl-3-(3-dimethylaminopropyl) carbodiimide (1.3 g, 7 mmol). Most of the solids dissolved. To this was added a solution of the hydrazine from the previous step (987 mg, 2.9 mmol) and DMAP (40 mg, 0.3 mmol) in $CH_2Cl_2$ (25 mL). The reaction was stirred under $N_2$ for 18 h, then washed with water. The aqueous phase was back extracted with ethyl acetate. The combined organics were dried over $MgSO_4$ and concentrated by rotary evaporation. The residue was chromatographed on silica gel eluting with 10 % methanol in chloroform. The pure fractions were combined and concentrated by rotary evaporation to give a white foam (1.58 g, 85 % yield).

The compound from the previous step (1.46 g, 2.28 mmol) was dissolved in THF (50 mL), and methanesulfonic acid (10 mL) was added. The reaction was stirred under $N_2$ for 16 h, at which time the volatiles were removed by rotary evaporation. Addition of 300 mL water to the residue precipitated the product, which was collected by vacuum filtration and washed with 150 mL water. The solid was air-dried and washed with ether (50 mL). The solid was suspended in a mix of 50 mL ethanol and 150 mL ether and sonicated for 30 min, and the solid was collected by vacuum filtration and dried at 200 mTorr and 30 °C for 16 h. This gave the title compound as a white solid (830 mg, 78 % yield). $^1$HNMR (DMSO-$d_6$) d 12.59 (s, 1H), 11.90 (s, 1H), 8.51 (s,1H), 8.15 (d, *J* = 8.7 Hz, 1H), 8.02 (s, 1H), 7.43 (d, *J* = 8.7 Hz, 1H), 7.32 (d, *J* = 8.7 Hz, 2H), 7.17 (d, *J* = 8.4 Hz, 2H), 6.15 (t, *J* = 5.7 Hz, 1H), 5.01 (s,2H), 3.45-3.35 (m, 2H), 3.26 (s, 3H), 3.36-3.20 (m, 2H). Anal. Calc'd for $C_{22}H_{20}ClN_5O_5$-$0.5H_2O$-$0.1CH_3SO_3H$; C, 54.34; H, 4.42; N, 14.34. Found C, 54.57, 54.56; H, 4.54, 4.77; N, 14.23, 14.15.

**Example 36:** 7-Chloro-4-hydroxy-2-(4-(3-(1-morpholinyl)propyl)amino-carboxamide)phenylmethyl-1,2,5,10-tetrahydropyridazino[4,5-*b*]quinoline-1,10-dione methanesulphonate.

The title compound was prepared by a method analogous to that of Example 35, but utilizing *N*-(3-aminopropyl)morpholine in place of 2-methoxyethylamine. $^1$HNMR (DMSO-$d_6$) d 12.60 (s,1H), 11.93 (s,1H), 9.54 (s,1H), 8.62 (s,1H), 8.15 (d, *J* = 8.7 Hz, 1H), 8.03 (s, 1H), 7.34 (d, *J* = 8.4 Hz, 2H), 7.17 (d, *J* = 8.7 Hz, 2H), 6.39 (t, *J* = 5.4 Hz, 1H), 5.01 (s, 2H), 3.97 (d, *J* = 11.7 Hz, 2H), 3.64 (t, *J* = 11.7 hz, 2H), 3.5-3.3 (m, 4H + $H_2O$), 3.2-2.9 (m, 4H), 2.36 (s, 3H), 1.86-1,79 (m, 2H).

**Example 37:** 7-Chloro-4-hydroxy-2-(4-(2-dimethylaminoethyl)methylamino-carboxamide)phenylmethyl-1,2,5,10-tetrahydropyridazino[4,5-*b*]quinoline-1,10-dione.

The title compound was prepared by a method analogous to that of Example 35, but using *N,N,N'*-trimethylethylenediamine in place of 2-methoxyethylamine.$^1$HNMR (DMSO-$d_6$) d 12.59 (s, 1H), 11.91 (s,1H), 9.21 (s, 1H), 8.37 (s, 1H), 8.15 (d, *J* = 8.7 Hz, 1H), 8.03 (s, 1H), 7.46-7.42 (m, 3H), 7.19 (d, *J* = 8.4 Hz, 2H), 5.03 (s, 2H), 3.62 (t, *J* = 6.3 Hz, 2H), 3.3-3.2 (m, 2H), 2.99 (s, 3H), 2.83 (d, *J* = 4.8 Hz, 6H).

**Tests for biological function:**

**Test A:** Inhibition of binding of [$^3$H]-MDL105.519:

Binding of compounds to the NMDA receptor glycine site may be assessed by measuring the ability of test compounds to inhibit the binding of tritiated MDL105,519 to brain membranes bearing the receptor.
Rat Brain Membranes: The rat brain membranes used in the experiments were obtained from Analytical Biological Services Inc., and were prepared substantially in accordance with the method of B.M. Baron *et al., J. Pharmacol. Exp. Ther.* 250, 162 (1989). Briefly, fresh brain tissue including cerebral cortex and hippocampus from male Sprague Dawley rats was homogenized in 0.32 M sucrose and centrifuged at low speed to separate cellular membranes from other cellular components. The membranes were then washed 3 times using deionized water, followed by treatment with 0.04% Triton X-100. Finally, membranes were washed six times in 50 mM Tris citrate buffer, pH 7.4, and frozen at -80 °C until use.
[$^3$H]MDL105,519 (72 Ci/mmol) was purchased from Amersham. Cold MDL105,519 was purchased from Sigma/RBI. Binding assays were performed substantially in accordance with the protocol of B.M. Baron *et al., J. Pharmacol. Exp. Ther*. 279, 62 (1996), as follows. On the day of the experiment, brain membranes were thawed at room temperature and suspended in 50 mM tris acetate buffer, pH 7.4 ("TAB"). Seventy-five micro grams per milliliter protein (by using the BioRad dye) were used for competition binding. The experiments were carried out using 96-well plates. Membranes were incubated with 20 $\mu$L of compounds of various concentrations and 1.2 nM [$^3$H]MDL105,519 for 30 minutes at room temperature in a total volume of 250 $\mu$L. Non specific binding was determined by using 100 $\mu$M of unlabeled MDL105,519. The unlabeled MDL105,519 and compounds were dissolved as 12.5 mM stock solutions in DMSO. Final DMSO concentration in each well was kept below 1%, which concentration was found not to alter the binding results. After incubation, unbound [$^3$H]MDL105,519 was removed by filtration onto GF/B Unifilter plates using a Packard harvester. Filters were washed four times with ice cold TAB (total of 1.2 mL buffer). The plates were dried overnight at room temperature and bound radioactivity was measured on a Packard TopCount after the addition of 45 $\mu$L per well of the MICROSCINT O.
Human Brain Membranes: Human brain membranes were obtained from Analytical Biological Services Inc., and assays were performed as described for rat membranes.
Data analysis: Data was analyzed using a Microsoft Excel spreadsheet and GraphPad Prizm software and potency of compounds is expressed as the Ki (nM).

**Test B:** Formalin test:

The Formalin test is an assay that assesses the capacity of a compound to inhibit formalin-induced nociceptive behaviors in rats (D. Dubuisson, *et al.*, *Pain* 4, 161-174 (1977); H. Wheeler-Aceto *et al.*, *Psychopharmacology* 104, 35-44 (1991); T.J. Coderre, *et al.*, *Pain* 54, 43-50 (1993)). In the test, two distinctive phases of formalin-induced behaviors are observed. A first phase response, caused by acute nociception to the noxious chemical (formalin) injected into the paw, occurs between zero and five minutes. A quiescent period of 5 to 15 min post injection follows. After the quiescent period a second phase response, caused by sensitization of the central neurons in the dorsal horn, occurs after 15 minutes and lasts up to 60 minutes. Sensitization of the central neurons in the spine augments a noxious afferent input and causes a stronger pain barrage to be transmitted to the brain. Therefore, inhibition of the second phase response indicates a central mechanism of drug action.
The procedure for the formalin test is as follows: male rats are placed in a plexiglass chamber and observed for 30-45 min. to observe their baseline activity. Animals are either pretreated with vehicle or with different doses of a test compound. Animals are dosed with vehicle or test compound three hours prior to injection of 0.05 mL of sterile 1% formalin under the dorsal skin of a hind paw. The number of paw flinches (responses) during the first phase (0-5 min.)

and the second phase (20-35 min.) are scored and recorded. Flinch response is compared with the mean score of a saline control group and calculated as percentage inhibition. The $ED_{50}$ is the dose of compound which produces 50% inhibition of nociceptive response in the first or second phase response. First phase responses may be inhibited by compounds that act peripherally and by compounds that act centrally. Second phase response are inhibited by centrally active compounds.

$$\% \text{ inhibition of nociceptive response} = 100 \times \frac{(\text{number of responses in vehicle group - number of responses in compound group})}{(\text{number of responses in vehicle group})}$$

Student's t-test was used for statistical analysis to determine the significance of compound effects. Data are reported as a dose that yielded a % inhibition of a response.

<u>**Test C:**</u> <u>Neuropathic pain model (Chronic Constriction Injury):</u>

The anti-hyperalgesic properties of a compound may be tested with the Chronic Constriction Injury ("CCI") model. The test is a model for neuropathic pain associated with nerve injuries that can arise directly from trauma and compression, or indirectly from a wide range of diseases such as infection, cancer, metabolic conditions, toxins, nutritional deficiencies, immunological dysfunction, and musculoskeletal changes. In the model a unilateral peripheral hyperalgesia is produced in rats by nerve ligation (G.J. Bennett, *et al*., *Pain* 33, 87-107 (1988)).

Procedurally, Sprague-Dawley rats (250-350 g) are anesthetized with sodium pentobarbital and the common sciatic nerve is exposed at the level of the mid thigh by blunt dissection through the biceps femoris. A section of nerve (about 7 mm), proximal to the sciatic trifucation, is freed of tissue and ligated at four positions with chromic gut suture. The suture is tied with about 1 mm spacing between ligatures. The incision is closed in layers and the animals are allowed to recuperate. Thermal hyperalgesia is measured using a paw-withdrawal test (K. Hargreaves, *et al., Pain* 32, 77-88 (1988)). To perform the test, animals are habituated on an elevated glass floor. A radiant heat source is aimed at the mid-plantar hindpaw (sciatic nerve territory) through the glass floor with a 20 second cut-off used to prevent injury to the skin. The latencies for the withdrawal reflex in both hind paws are recorded.

Injured paws with ligated nerves show shorter paw withdrawal latencies compared to the uninjured or sham operated paws. Responses to test compounds are evaluated at different times after oral administration to determine the onset and duration of compound effect. When performing the test, groups of CCI rats receive either vehicle or the test compound orally three times daily for 5 days. Paw withdrawal latencies are measured each day 10 min before and 2 or 3 hr. after the first daily dose. Compound efficacy is expressed as mean percentage decrease of hyperalgesia compared to that of vehicle-treated animals, calculated as follows:

$$\frac{(\text{Mean of vehicle group - Mean of compound group})}{(\text{Mean of vehicle group}).} \times 100$$

Data analysis was performed by the multiple means comparison test (Dunnett's test) and results are expressed and compound potencies are expressed as the MED (minimum effective dose), in mg/Kg/day, that yields a percent (%) decrease in hyperalgesia that is statistically significant.

Table 1 shows the results from Tests A, B and C for certain compounds of the invention. Where no data is provided in the table, the test was not performed.

Table 1

| Example | Test A Ki (nM) | Test B First phase Dose (%Inh.) | Test B Second phase Dose (%Inh.) | Test C MED (%Inh.) |
|---|---|---|---|---|
| Ex. 1 | 39.9 | 200 (43%) | 200 (54%) | 15 (82%) |
| Ex. 2 | 55 | | | |
| Ex. 3 | 153 | 200 (33%) | 200 (-18%) | 30 (96%) |

Table continued

| Example | Test A Ki (nM) | Test B First phase Dose (%Inh.) | Test B Second phase Dose (%Inh.) | Test C MED (%Inh.) |
|---|---|---|---|---|
| Ex. 5 | 117 | | | |
| Ex. 6 | 67.9 | | | |
| Ex. 6 | 62.5 | | | |
| Ex. 7 | 111 | | | |
| Ex. 8 | 35 | | | |
| Ex. 9 | 91 | | | |
| Ex. 10 | 96 | | | |
| Ex. 11 | 59.5 | | | 15 (80%) |
| Ex. 12 | 30.5 | | | |
| Ex. 13 | 67.5 | | | |
| Ex. 14 | 112 | | | 15 (14%) |
| Ex. 15 | 15.6 | | | |
| Ex. 16 | 86 | | | |
| Ex. 17 | 110 | | | |
| Ex. 18 | 79.9 | | | |
| Ex. 19 | 33.3 | | | |
| Ex. 20 | 199 | | | |
| Ex. 21 | 1.0 | | | |
| Ex. 22 | 974 | | | |
| Ex. 23 | 141 | | | |
| Ex. 24 | 65 | | | |
| Ex. 25 | 126 | | | |
| Ex. 27 | 191 | | | |
| Ex. 28 | 308 | | | |
| Ex. 29 | 88 | | | |
| Ex. 30 | 98 | | | |
| EX. 31 | 41 | | | |

## Claims

**1.** A compound seleaed from:

7-Chloro-4-hydroxy-2-(2,4,6-trimethylphenyl)-1,2,5.10-tetrahydropyridazino[4,5-*b*]quinoline-1,10-dione;
7,9-Dichloro-4-hydroxy-2-(4-methoxy-2-methylphenyl)-1,2,5,10-tetrahydropyridazino[4,5-*b*]quinoline-1,10-dione;
Methyl 4-[(7-chloro-4-hydroxy-1,10-dioxo-2,5-dihydropyridazino[4,5-b]quinolin-2-yl)methyl]benzoate;
7-Chloro-4-hydroxy-2-(4-*N*-furan-2-ylmethyl)benzamidylmethyl-1,2,5,10-tetrahydropyridazino[4,5-*b*]quinoline-1,10-dione;
7-Chloro-4-hydroxy-2-(4-dimethylaminobenzyl)-1,2,5,10-tetrahydropyridazino[4,5-*b*]quinoline-1,10-dione;
7-Chloro-4-hydroxy-2-(benzimidazol-5-ylmethyl)-1,2,5,10-tetrahydropyridazino[4,5-*b*]quinoline-1,10-dione;
7-Chloro-4-hydroxy-2-(4-hydroxy-3-methyl-benzyl)-1,2,5,10-tetrahydropyridazino[4,5-*b*]quinoline-1,10-dione;

7-Chloro-4,10-dihydroxy-2-[(2-methylthiophenyl)methyl]-2-hydropyridazino[4,5-*b*]quinoline-1-one;
7-Chloro- 2-(4-(2,6-dimethoxy) methylbenzoate)- 4-hydroxy- 1,2,5,10-tetrahydropyridazino [4,5-*b*] quinoline-1,10-dione;
Methyl 3-[(7-chloro-4-hydroxy-1,10-dioxo-2,5-dihydropyridazino[4,5-*b*]quinolin-2-yl)mexhyl]benzoate;
7-Chloro-4-hydroxy-2-(4-dimethylaminocarboxamide)phenylmetbyl-1,2,5,10-tetrahydropyridazino[4,5-*b*]quinoline-1,10-dione;
7-Chloro-4-hydroxy-2-(4-(*tert*-buryl)aminocarboxamide)phenylmethyl-1,2,5,10-tetrahydropyridazino[4,5-*b*]quinoline-1,10-dione;
7-Chloro- 4-hydroxy- 2-(4-pyrrolidinylcarboxamide) phenylmethyl- 1,2,5,10-tetrahydropyridazino [4,5-*b*] quinoline-1,10-dione;
7-Chloro- 4-hydroxy- 2-(3-pyrrolidinylcarboxamide) phenylmethyl- 1,2,5,10-tetrahydropyridazino [4,5-*b*] quinoline-1,10-dione;
7-Chloro-2-(3-aminomethylphenyl)-4-hydroxy-1,2,5,10-tetrahydropyridazino[4,5-*b*]quinoline-1,10-dione;
7-Chloro-4-hydroxy-2-(4-phenylaminocarboxamide)phenylmethyl-1,2,5,10-tetrahydropyridazino[4,5-*b*]quinoline-1,10-dione;
7-Chloro-4-hydroxy-2-(3-phenylaminocarboxamide)phenylmethyl-1,2,5,10-tetrahydropyridazino[4,5-*b*]quinoline-1,10-dione;
7-Chloro-4-hydroxy-2-(4-iodophenylmethyl)-1,2,5,10-tetrahydropyridazino[4,5-*b*]quinoline-1,10-dione;
7-Chloro-4-hydroxy-2-(4-(3-proparginol)phenyl)-1,2,5,10-tetrahydropyridazino[4,5-*b*]quinoline-1,10-dione;
{3-[(7-Chloro- 4-hydroxy- 1,10-dioxo (2,5-dihydropyridazino [4,5-b] quinolin- 2-yl)) methyl] phenyl} -N-methoxy-N-methylcarboxamide;
{3-[(7-Chloro-4-hydroxy-1,10-dioxo(2,5-dihydropyridazino [4,5-b] quinolin-2-yl)) methyl] phenyl} -N-methylcarboxamide;
{3-[(7-Chloro-4-hydroxy-1,10-dioxo(2,5-dihydropyridazino[4,5-b]quinolin-2-yl))methyl]phenyl}-N-2-methoxyethyl)carboxamide;
{3-[(7-Chloro- 4-hydroxy- 1,10-dioxo (2,5-dihydropyridazino [4,5-b] quinolin- 2-yl)) methyl] phenyl} -N, N-bis (2-ethoxyethyl)carboxamide, and
7-Chloro-4-hydroxy-2-{[3-(piperazinylcarbonyl)phenyl]methyl}-1,2,5,10-tetrahydropyridazino[4,5-*b*]quinoline-1,10-dione,

or pharmaceutically-acceptable salts thereof.

**2.** A pharmaceutical composition comprising treatment with a pain-ameliorating effective amount of a compound in accordance with claim 1, together with a pharmaceutically-acceptable excipient or diluent

**3.** Use of a compound in accordance with claim 1 in the manufacture of a medicament for the treatment of neuropathic pain.

**4.** A method for making compounds according to claim 1;

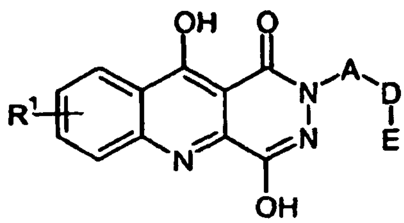

I

wherein:

$R^1$ is halo;
A is $(CH_2)_n$ where n is 1;
D-E is a moiety according to formula II;

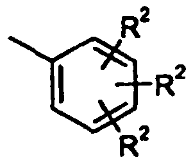

II;

$R^2$ at each occurrence is selected from hydrogen, hydroxyl, halo, $C_{1-4}$alkyl, $C_{1-4}$alkoxy, hydroxy$C_{2-6}$alkylnyl, $C_{1-3}$alkylOC(O)O, $C_{1-3}$alkylS(O)$_m$ where m has a value selected from 0, 1 or 2, benzimidazolyl, $NR^3R^4$, C(O) $NR^3R^4$ and

NHC(O)$NR^3R^4$ where $R^3$ and $R^4$ at each occurrence are independently selected from hydrogen, $C_{1-4}$alkyl, $C_{1-4}$alkoxy, $(CH_2)_nOC_{1-4}$alkyl where n is selected from

1, 2, 3 or 4, $C_{1-3}$alkylfuranyl, cyclohexyl and phenyl, or the group $NR^3R^4$ is selected from morpholinyl, piperazinyl or pyrrolidinyl, and where in any compound of formula I at least one R moiety is other than hydrogen;

said method comprising:

a) preparing a Boc-protected hydrazine by reacting a ketone or an aldehyde, according to one of the procedures shown in the following scheme, or alternatively by reacting an alkyl halide as shown in the following scheme:

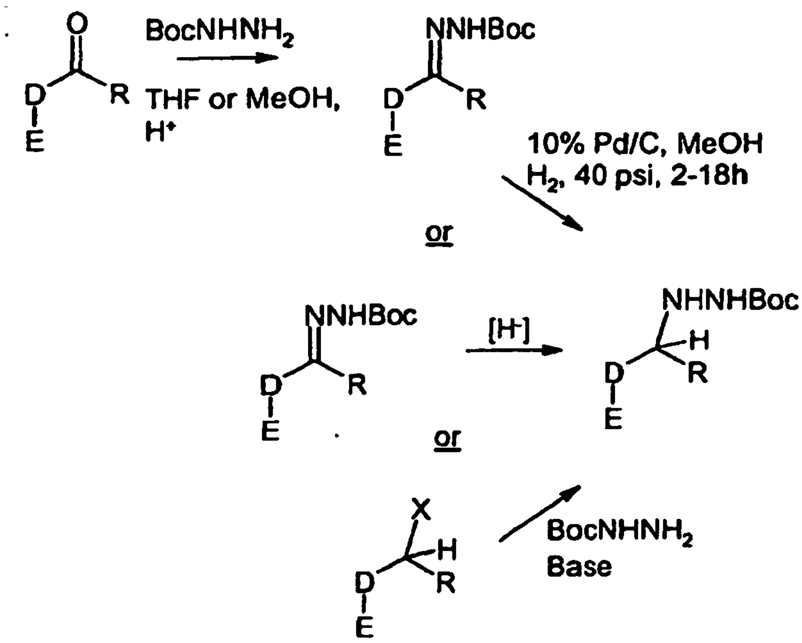


X = Cl, Br or OMs; R = H or alkyl.

alternatively, a hydrazine may be synthesized from an aromatic aldehyde by the procedure shown in the following scheme:

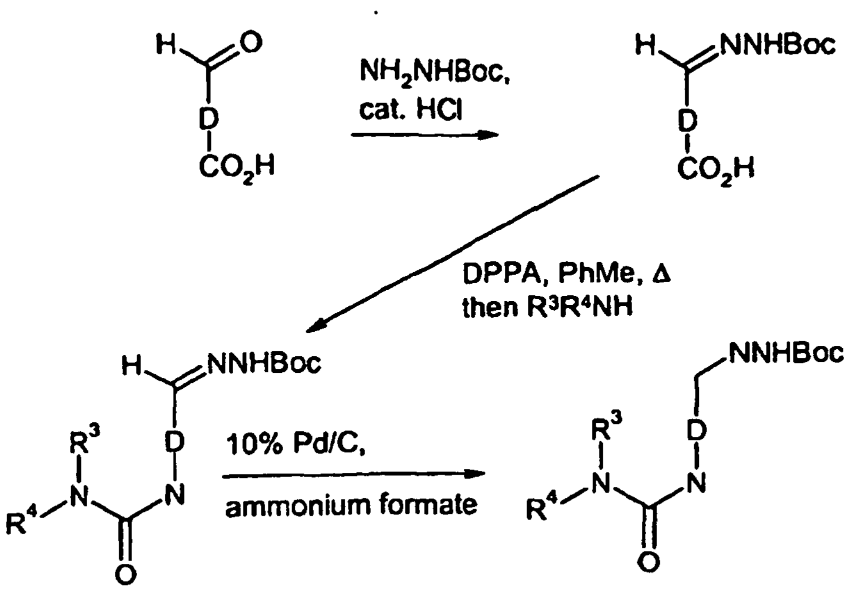


b) coupling said Boc-protected hydrazine and cyclizing the product according to the process of the following scheme to form a compound according to formula I:

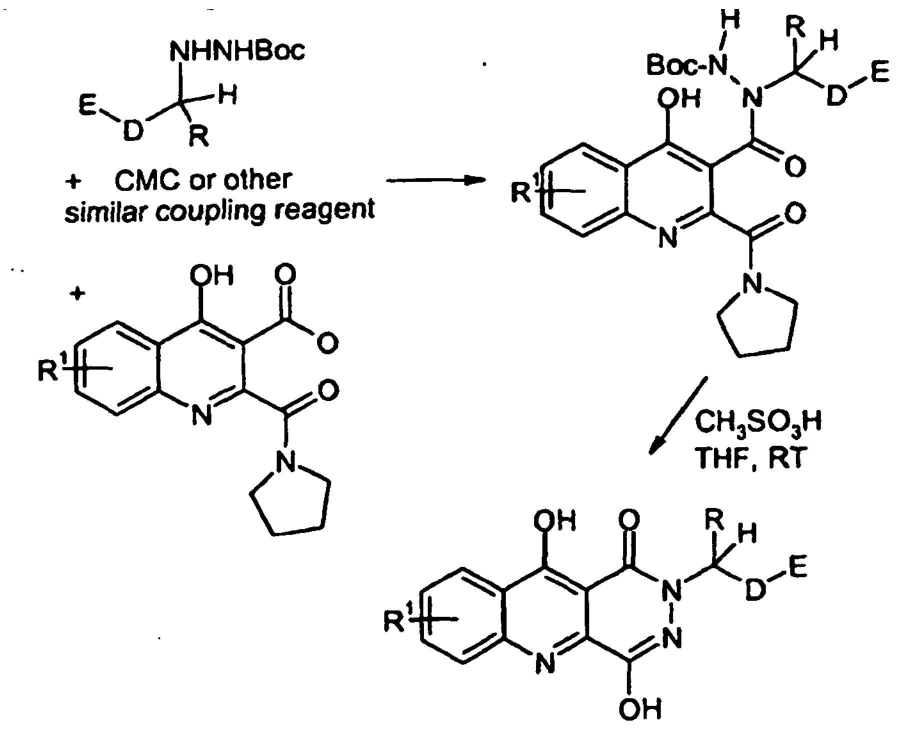


wherein:
CMC is 1-cyclohexyl-3-(2-morpholinoethyl)carbodiimide metho-p-toluenesulfonate;
the "R/H/D-E" group is the "-A-D-E" moiety of formula I, and
throughout the foregoing process $R^1$ is as defined for formula I.

## Revendications

**1.** Composé choisi parmi :

La 7-chloro-4-hydroxy-2-(2,4,6-triméthylphényl)-1,2,5,10-tétrahydropyridazino[4,5-*b*]quinoléine-1,10-dione ;
la 7,9-dichloro-4-hydroxy-2-(4-méthoxy-2-méthylphényl)-1,2,5,10-tétrahydropyridazino[4,5-*b*]quinoléine-1,10-dione ;
le méthyl-4-[(7-chloro-4-hydroxy-1,10-dioxo-2,5-dihydropyridazino [4,5-*b*]quinoléine-2-yl)méthyl]benzoate ;
La 7-chloro-4-hydroxy-2-(4-N-furane-2-ylméthyl)benzamidylméthyl-1,2,5,10-tétrahydropyridazino[4,5-b]quinoléine-1,10-dione ;
la 7-chloro-4-hydroxy-2-(4-diméthylaminobenzyl)-1,2,5,10-tétrahydropyridazino[4,5-*b*]quinoléine]-1,10-dione ;
la 7-chloro-4-hydroxy-2-(benzimidazol-5-ylméthyl)-1,2,5,10-tétrahydropyridazino[4,5-*b*]quinoléine-1,10-dione ;
la 7-chloro-4-hydroxy-2-(4-hydroxy-3-méthyl-benzyl)-1,2,5,10-tétrahydropyridazino[4,5-*b*]quinoléine-1,10-dione ;
la 7-chloro-9,10-dihydroxy-2-[(2-méthylthiophényl)méthyl]-2-hydropyridazino [4,5-*b*]quinoléine-1-one ;
la 7-chloro-2-(4-(2,6-diméthoxy)méthylbenzoate)-4-hydroxy 1,2,5,10-tétrahydropyridazino[4,5-*b*]quinoléine-1,10-dione ;
le méthyl 3-[(7-chloro-4-hydroxy-1,10-dioxo-2,5-dihydropyridazino[4,5-*b*]quinoléine-2-yl)méthyl]benzoate ;
la 7-chloro-4-hydroxy-2-(4-diméthylaminocarboxamide)phénylméthyl-1,2,5,10-tétrahydropyridazino[4,5-b]quinoléine-1,10-dione ;
la 7-chloro-4-hydroxy-2-(4-(*tert*-butyl) aminocarboxamide)phénylméthyl-1,2,5,10-tétrahydropyridazino[4,5-*b*] quinoléine-1,10-dione ;
la 7-chloro-9-hydroxy-2-(4-pyrrolidinylcarboxamide)phénylméthyl -1,2,5,10-tétrahydropyridazino[4,5-*b*]quinoléine-1,10-dione ;
la 7-chloro-4-hydroxy-2-(3-pyrrolidinylcarboxamide)phénylméthyl -1,2,5,10-tétrahydropyridazino[4,5-*b*]quinoléine-1,10-dione ;
la 7-chloro-2-(3-aminométhylphényl)-4-hydroxy-1,2,5,10-tétrahydropyridazino[4,5-b]quinoléine-1,10-dione ;
la 7-chloro-4-hydroxy-2-(4-phénylaminocarboxamide)phénylméthyl-1,2,5,10-tétrahydropyridazino[4,5-*b*]quinoléine-1,10-dione ;
la 7-chloro-4-hydroxy-2-(3-phénylaminocarboxamide)phénylméthyl-1,2,5,10-tétrahydropyridazino[4,5-b]quinoléine-1,10-dione ;
la 7-chloro-4-hydroxy-2-(4-iodophénylméthyl)-1,2,5,10-tétrahydropyridazino[4,5-b]quinoléine-1,10-dione ;
la 7-chloro-4-hydroxy-2-(4-(3-proparginol)phényl) -1,2,5,10-tétrahydropyridazino[4,5-*b*]quinoléine-1,10-dione ;
le {3-[(7-chloro-4-hydroxy-1,10-dioxo(2,5-dihydropyridazino[4,5-b]quinoléin-2-yl))méthyl]phényl}-N-méthoxy-N-méthylcarboxamide ;
le {3-[(7-chloro-4-hydroxy-1,10-dioxo(2,5-dihydropyridazino[4,5-b]quinoléin-2-yl))méthyl]phényl}-N-méthylcarboxamide ;
le {3-[(7-chloro-4-hydroxy-1,10-dioxo(2,5-dihydropyridazino[4,5-b]quinoléin-2-yl))méthyl]phényl}-N-2-méthoxyéthyl)carboxamide ;
le {3-[(7-chloro-4-hydroxy-1,10-dioxo(2,5-dihydropyridazino[4,5-b]quinoléin-2-yl))méthyl]phényl}-N,N-bis(2-éthoxyéthyl)carboxamide ; et
la 7-chloro-4-hydroxy-2-{[3-pipérazinylcarbonyl)phényl]méthyl}-1,2,5,10-tétrahydropyridazino[4,5-*b*]quinoléine-1,10-dione, ou leurs sels pharmaceutiquement acceptables.

**2.** Composition pharmaceutique comprenant le traitement avec une quantité efficace, soulageant la douleur, d'un composé selon la revendication 1, conjointement avec un excipient ou un diluant pharmaceutiquement acceptable.

**3.** Utilisation d'un composé selon la revendication 1 pour la fabrication d'un médicament destiné au traitement de la douleur névropathique.

**4.** Procédé de production de composés selon la revendication 1 :

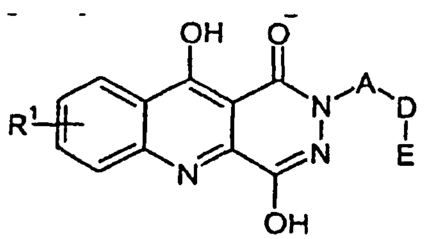

I

dans laquelle :

$R^1$ est un halogéno ;
A est $(CH_2)_n$ dans lequel n vaut 1 ;
D-E est un fragment selon la formule II ;

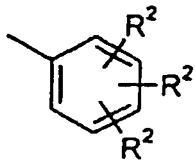

II;

$R^2$, dans chaque cas, est choisi parmi un hydrogène, un hydroxyl, un halogéno, un alkyle en $C_{1-4}$, un alcoxy en $C_{1-4}$, un hydroxy$C_{2-6}$alcynyle, un $C_{1-3}$alkyl-OC(O)O, un $C_{1-3}$-alkyl-$S(O)_m$ dans lequel m présente une valeur choisie parmi 0, 1 ou 2, un benzimidazolyle, un $NR^3R^4$, un $C(O)NR^3R^4$ et un $NHC(O)NR^3R^4$ dans lesquels $R^3$ et $R^4$, dans chaque cas, sont choisis indépendamment parmi un hydrogène, un alkyle en $C_{1-4}$, un alcoxy en $C_{1-4}$, un $(CH_2)_nO$-$C_{1-4}$-alkyle dans lequel n est choisi parmi 1,2,3 ou 4, un $C_{1-3}$-alkylfuranyle, un cyclohexyle et un phényle, ou le groupe $NR^3R^4$ est choisi parmi un morpholinyle, un pipérazinyle ou un pyrrolidinyle, et où dans tout composé de formule I, au moins un fragment
$R^2$ est autre qu'un hydrogène ;

ledit procédé comprenant :

a) la préparation d'une hydrazine protégée par Boc par réaction d'une cétone ou d'un aldéhyde, conformément à l'un des modes opératoires illustrés dans le schéma suivant, ou en variante par réaction d'un halogénure d'alkyle tel qu'illustré dans le schéma suivant :

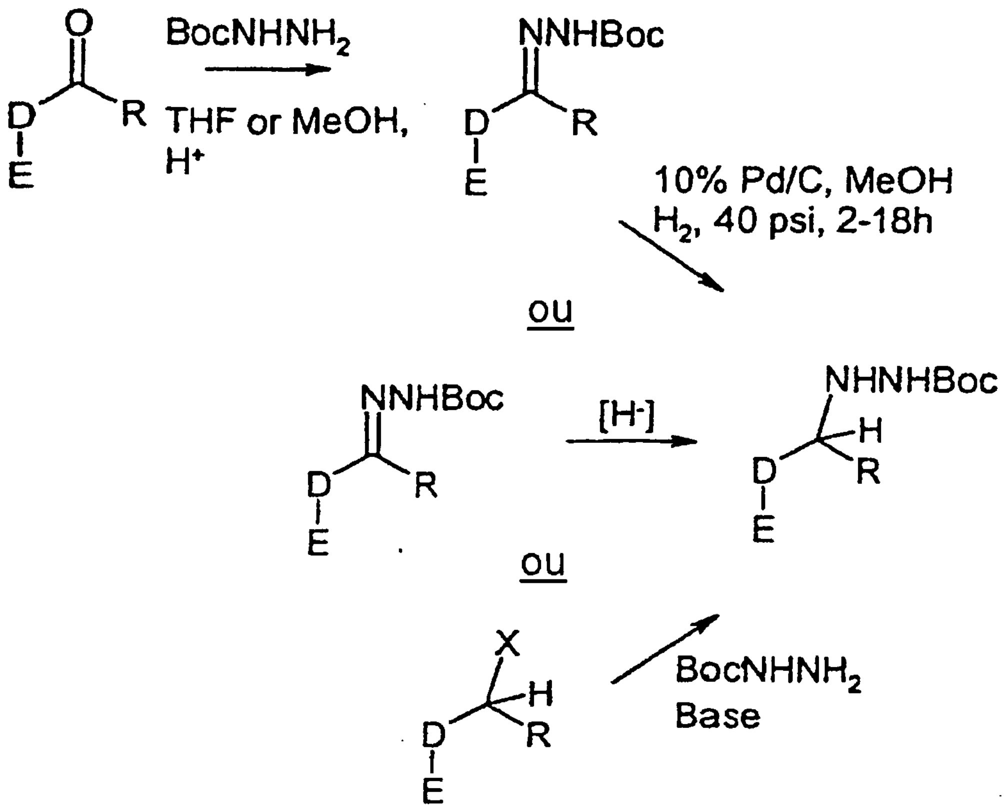


X = Cl, Br ou OMs; R = H ou alkyl.

En variante, une hydrazine peut être synthétisée à partir d'un aldéhyde aromatique par le mode opératoire illustré dans le schéma suivant :

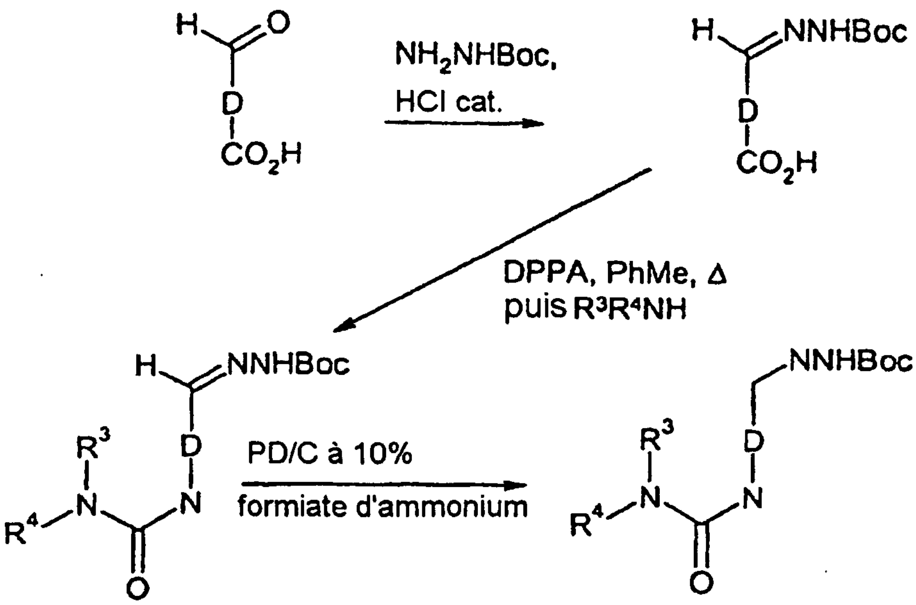


b) le couplage de ladite hydrazine protégée par Boc et la cyclisation du produit conformément au procédé du schéma suivant en vue de former un composé selon la formule I :

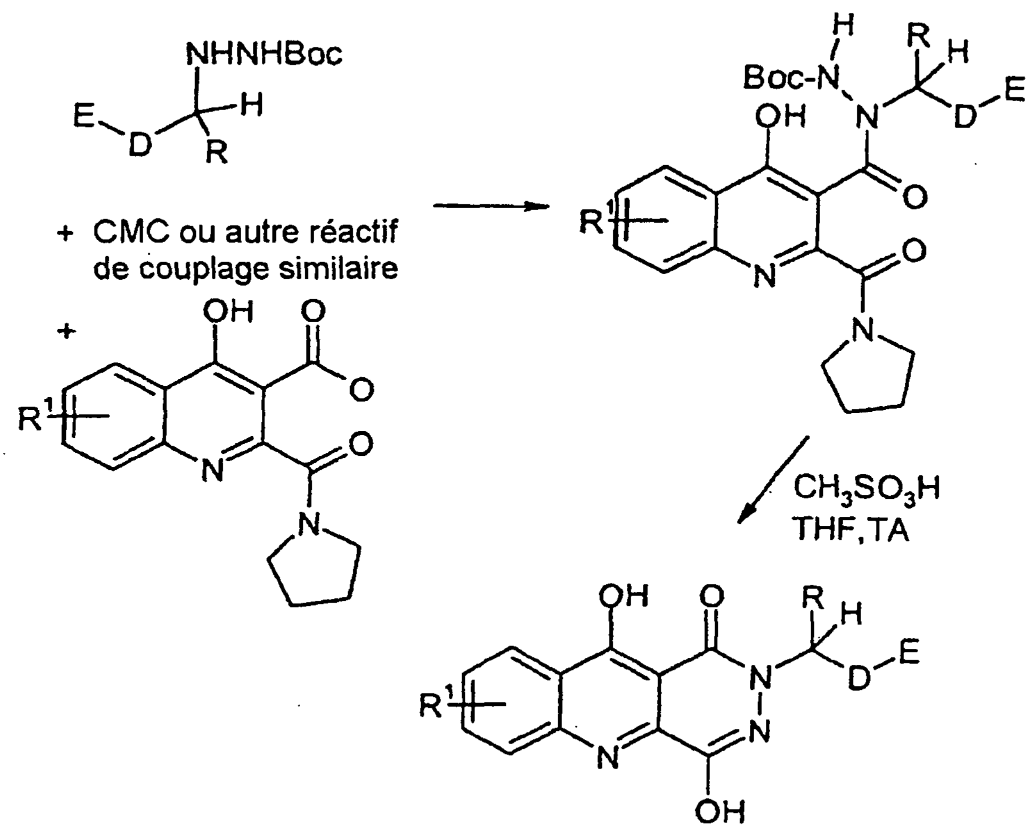


dans lequel :

CMC est le 1-cyclohexyl-3-(2-morpholinoéthyl)carbodiimide-métho-p-toluènesulfonate ; Le groupe « R/H/D-E » est le fragment « -A-D-E » de formule I, et dans l'ensemble du procédé précédent, $R^1$ est tel que défini par la formule I.

## Patentansprüche

**1.** Verbindungen, ausgewählt aus:

7-Chlor-4-hydroxy-2-(2,4,6-trimethylphenyl)-1,2,5,10-tetrahydropyridazino[4,5-b]chinolin-1,10-dion;
7,9-Dichlor-4-hydroxy-2-(4-methoxy-2-methylphenyl)-1,2,5,10-tetrahydropyridazino[4,5-b]chinolin-1,10-dion;
4-[(7-Chlor-4-hydroxy-1,10-dioxo-2,5-dihydropyridazino[4,5-b]chinolin-2-yl)methyl]benzoesäuremethylester;
7-Chlor- 4-hydroxy- 2-(4-N-furan- 2-ylmethyl) benzamidylmethyl- 1,2,5,10-tetrahydropyridazino [4,5-b] chinolin-1,10-dion;
7-Chlor-4-hydroxy-2-(4-dimethylaminobenzyl)-1,2,5,10-tetrahydropyridazino[4,5-b]chinolin-1,10-dion;
7-Chlor-4-hydroxy-2-(benzimidazol-5-ylmethyl)-1,2,5,10-tetrahydropyridazino[4,5-b]chinolin-1,10-dion;
7-Chlor-4-hydroxy-2-(4-hydroxy-3-methylbenzyl)-1,2,5,10-tetrahydropyridazino[4,5-b]chinolin-1,10-dion;
7-Chlor-4,10-dihydroxy-2-[(2-methylthiophenyl)methyl]-2-hydropyridazino[4,5-b]chinolin-1-on;
7-Chlor-2-(4-(2,6-dimethoxy) methylbenzoat)-4-hydroxy-1,2,5,10-tetrahydropyridazino[4,5-b]chinolin-1,10-dion;
3-[(7-Chlor-4-hydroxy-1,10-dioxo-2,5-dihydropyridazino[4,5-b]chinolin-2-yl)methyl]benzoesäuremethylester;
7-Chlor-4-hydroxy-2-(4-dimethylaminocarboxamid)phenylmethyl-1,2,5,10-tetrahydropyridazino[4,5-b]chinolin-1,10-dion;
7-Chlor- 4-hydroxy- 2-(4-(tert.-butyl) aminocarboxamid) phenylmethyl- 1,2,5,10-tetrahydropyridazino [4,5-b] chinolin-1,10-dion;
7-Chlor- 4-hydroxy- 2-(4-pyrrolidinylcarboxamid) phenylmethyl- 1,2,5,10-tetrahydropyridazino [4,5-b] chinolin-1,10-dion;

7-Chlor- 4-hydroxy- 2-(3-pyrrolidinylcarboxamid) phenylmethyl- 1,2,5,10-tetrahydropyridazino [4,5-b] chinolin-1,10-dion;
7-Chlor-2-(3-aminomethylphenyl)-4-hydroxy-1,2,5,10-tetrahydropyridazino[4,5-b]chinolin-1,10-dion;
7-Chlor- 4-hydroxy- 2-(4-phenylaminocarboxamid) phenylmethyl- 1,2,5,10-tetrahydropyridazino [4,5-b] chinolin-1,10-dion;
7-Chlor- 4-hydroxy- 2-(3-phenylaminocarboxamid) phenylmethyl- 1,2,5,10-tetrahydropyridazino [4,5-b] chinolin-1,10-dion;
7-Chlor-4-hydroxy-2-(4-iodphenylmethyl)-1,2,5,10-tetrahydropyridazino[4,5-b]chinolin-1,10-dion;
7-Chlor-4-hydroxy-2-(4-(3-proparginol)phenyl)-1,2,5,10-tetrahydropyridazino[4,5-b]chinolin-1,10-dion;
{3-[(7-Chlor- 4-hydroxy- 1,10-dioxo (2,5-dihydropyridazino [4,5-b] chinolin- 2-yl)) methyl] phenyl} -N-methoxy-N-methylcarboxamid;
{3-[(7-Chlor- 4-hydroxy- 1,10-dioxo (2,5-dihydropyridazino [4,5-b] chinolin- 2-yl)) methyl] phenyl} -N-methylcarboxamid;
{3-[(7-Chlor- 4-hydroxy- 1,10-dioxo (2,5-dihydropyridazino [4,5-b] chinolin- 2-yl)) methyl] phenyl} -N- 2-methoxyethyl)carboxamid;
{3-[(7-Chlor- 4-hydroxy- 1,10-dioxo (2,5-dihydropyridazino [4,5-b] chinolin- 2-yl)) methyl] phenyl} -N, N-bis (2-ethoxyethyl)carboxamid, und
7-Chlor- 4-hydroxy- { [3-(piperazinylcarbonyl) phenyl] methyl}- 1,2,5,10-tetrahydropyridazino [4,5-b] chinolin-1,10-dion, und deren pharmazeutisch annehmbaren Salzen.

**2.** Pharmazeutische Zusammensetzung, umfassend eine Behandlung mit einer schmerzlindernd wirksamen Menge einer Verbindung nach Anspruch 1 zusammen mit einem pharmazeutisch annehmbaren Hilfsstoff oder Verdünnungsmittel.

**3.** Verwendung einer Verbindung nach Anspruch 1 bei der Herstellung eines Medikaments zur Behandlung von neuropathischen Schmerzen.

**4.** Verfahren zur Herstellung von Verbindungen nach Anspruch 1

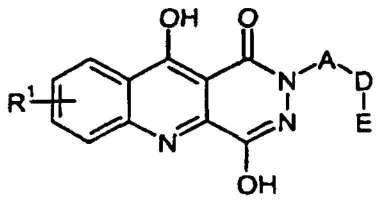

I

wobei:

$R^1$ für Halogen steht;
A für $(CH_2)_n$ steht, wobei n für 1 steht;
D-E für eine Einheit gemäß Formel II steht;

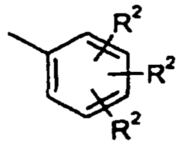

II;

$R^2$ jeweils aus Wasserstoff, Hydroxy, Halogen, $C_{1-4}$-Alkyl, $C_{1-4}$-Alkoxy, Hydroxy-$C_{2-6}$-Alkylnyl, $C_{1-3}$-Alkyl-OC(O)O, $C_{1-3}$-Alkyl-S(O)$_m$, wobei m einen aus 0, 1 oder 2 ausgewählten Wert aufweist, Benzimidazolyl, $NR^3R^4$, $C(O)NR^3R^4$ und $NHC(O)NR^3R^4$ ausgewählt ist, wobei $R^3$ und $R^4$ jeweils unabhängig voneinander aus Was-

serstoff, $C_{1-4}$-Alkyl, $C_{1-4}$-Alkoxy, $(CH_2)_nO$-$C_{1-4}$-Alkyl, wobei n aus 1, 2, 3 oder 4 ausgewählt ist, $C_{1-3}$-Alkyfuranyl, Cyclohexyl und Phenyl ausgewählt sind oder die Gruppe $NR^3R^4$ aus Morpholinyl, Piperazinyl oder Pyrrolidinyl ausgewählt ist, und
wobei in einer Verbindung der Formel I wenigstens eine $R^2$-Einheit nicht für Wasserstoff steht;

wobei man bei dem Verfahren:

a) ein Boc-geschütztes Hydrazin durch Umsetzung eines Ketons oder eines Aldehyds nach einer der im folgenden Schema gezeigten Vorschriften oder alternativ dazu durch Umsetzung eines Alkylhalogenids wie im folgenden Schema gezeigt:

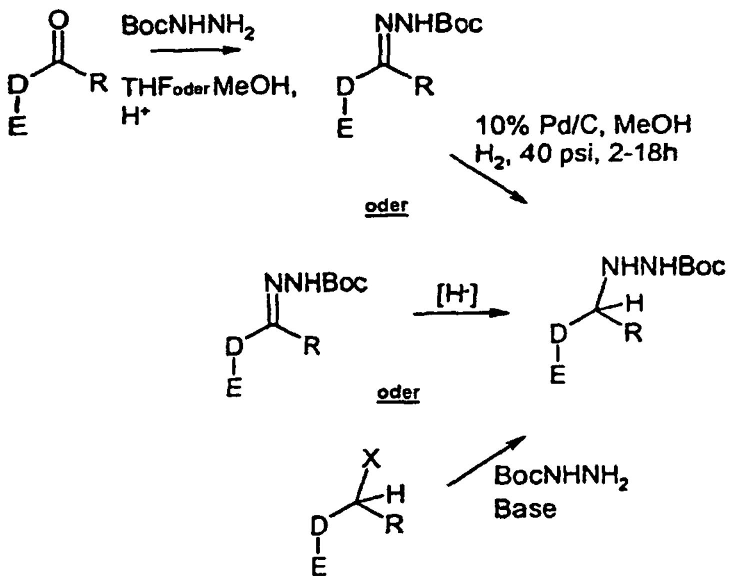


X = Cl, Br oder OMs; R = H oder Alkyl
herstellt oder alternativ dazu durch die im folgenden Schema gezeigte Vorschrift ein Hydrazin aus einem aromatischen Aldehyd synthetisieren kann

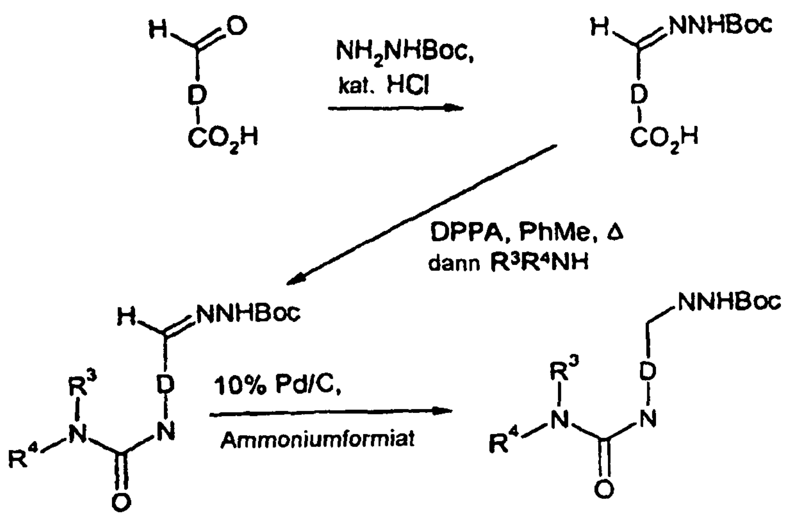


b) das Boc-geschützte Hydrazin kuppelt und das Produkt gemäß dem Verfahren des folgenden Schemas zur

Bildung einer Verbindung der Formel I cyclisiert:

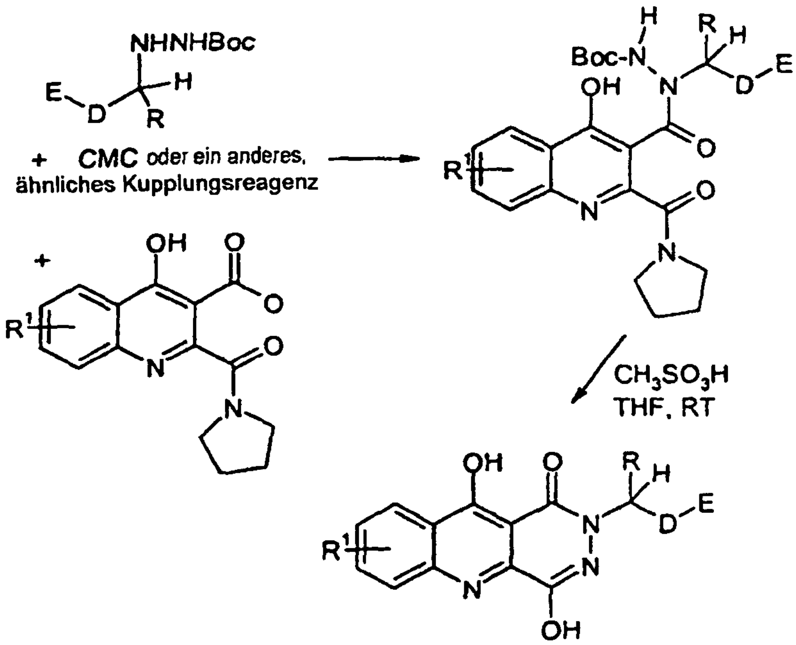


wobei:

CMC für 1-Cyclohexyl-3-(2-morpholinoethyl)carbodiimid-metho-p-toluolsulfonat steht; es sich bei der "R/H/D-E"-Gruppe um die "-A-D-E"-Einheit der Formel I handelt und im gesamten obigen Verfahren $R^1$ wie Formel I definiert ist.